# EUROPEAN PATENT APPLICATION

(11) **EP 4 844 006 A1**
(43) Date of publication of application: **25.11.2026**
(21) Application number: 25741561.2
(22) Date of filing: 16.01.2025
(51) Int. Cl.: C07K 16/40, C12N 15/63, C12N 15/64, A61K 39/395, A61P 13/12

(54) **ANTI-MASP2 ANTIBODY OR ANTIGEN-BINDING FRAGMENT AND USE THEREOF**

(30) Priority: 17.01.2024 CN 202410069597; 31.05.2024 CN 202410705582
(71) Applicant: Salubris (Chengdu) Biotech Co., Ltd., Chengdu, Sichuan 610041 (CN); Shenzhen Salubris Pharmaceuticals Co., Ltd., Shenzhen, Guangdong 518017 (CN)
(72) Inventor: MOU, Zongchun, Chengdu, Sichuan 610041 (CN); WEI, Wen, Chengdu, Sichuan 610041 (CN); ZHANG, Peng, Chengdu, Sichuan 610041 (CN)
(74) Representative: karo IP
(86) International application number: PCT/CN2025/072651
(87) International publication number: WO 2025/153000

(57) **Abstract**

The present disclosure provides an anti-MASP2 antibody and an antigen-binding fragment thereof, and further provides a polynucleotide encoding the antibody, a vector and a host cell for expressing the antibody, a pharmaceutical composition comprising the antibody, and a method and a pharmaceutical use of the antibody for treating MASP2-related diseases.

## Description

### TECHNICAL FIELD

The present disclosure belongs to the field of biomedical technology, and specifically relates to an anti-MASP2 antibody or an antigen-binding fragment thereof, a pharmaceutical composition and use thereof.

### BACKGROUND ART

The complement system is primarily activated through three pathways: classical, alternative, and lectin. Mannan-binding lectin-associated serine protease 2 (MASP2) is a key enzyme of the complement lectin pathway. Its encoding gene is located on chromosome 1p36 and encodes a 76 kDa serine protease MASP2 (long isoform) highly expressed in the liver and a 19 kDa alternative splicing product Map19 (short isoform) present in plasma. The MASP2 encoding gene consists of 12 exons, wherein exon 2 and exon 3 encode the CUB1 domain, exon 4 encodes the EGFL domain, exon 6 and exon 7 encode the CUB2 domain, exons 8 to 11 respectively encode two CCP domains, and exon 12 encodes the peptide activation region, SP domain, and C-terminal mRNA untranslated region of MASP2. The mature zymogen form of MASP2 consists of 686 amino acid residues. Once lectin recognizes and binds to a pathogen, the zymogen form of MASP2 is cleaved between CCP2 and SP, forming an active form of two polypeptide chains linked by disulfide bonds. The peptide activation region and SP domain of activated MASP2 can effectively bind and cleave C2, and with the assistance of the CCP domain, bind and cleave C4 to initiate subsequent cascade reactions, forming C4bC2a (i.e., C3 convertase), ultimately promoting the formation of the membrane attack complex composed of C5-C9, causing lysis of the pathogen, thereby resisting pathogenic microorganisms such as bacteria, yeasts, and viruses.

Clinical experiments and research evidence indicate that various diseases are associated with abnormal activation of the complement system, for example, related to abnormal or overexpression of MASP2, including IgA nephropathy (Lafayette, Richard A et al. Kidney international reports vol. 5,11 2032-2041. 13 Aug. 2020), thrombotic microangiopathy (Elhadad, S et al. Clinical and experimental immunology vol. 203,1 (2021): 96-104), systemic lupus erythematosus (Xu, Wang-Dong et al. Journal of cellular and molecular medicine vol. 24,18 (2020): 10432-10443), membranous nephropathy, lupus nephritis (Cai Y et al. PLoS One, 2013,8(4): e62465), transplant rejection, antiphospholipid syndrome (Boldt AB, et al. Hum Immunol, 2011, 72(9): 753-760), rheumatoid arthritis, hemolytic uremic syndrome, hereditary angioedema (Krarup A et al. PLoS One, 2007, 2(7): e623), myocardial infarction (Zhang M, et al. Int J Cardiol, 2013, 166(2): 499-504), etc.

Currently, various anti-MASP2 antibodies are disclosed in patent documents such as CN103687620B, WO2022228364A1, CN114634575A, and CN116284412A. However, there is still a lack of antibodies targeting MASP2 with new structures in the market, and further improving the affinity and biological activity of anti-MASP2 antibodies remains a technical problem to be solved in the art.

### SUMMARY

In one aspect, the present disclosure provides an anti-MASP2 antibody or an antigen-binding fragment thereof. The anti-MASP2 antibody or antigen-binding fragment thereof can specifically bind to MASP2 protein with high affinity, has no cross-reaction with other complement components (e.g., C1s, C1r, MASP1, and MASP3), and effectively inhibits the activation of the downstream complement pathway mediated by MASP2.

In one aspect, the present disclosure provides an anti-MASP2 antibody or an antigen-binding fragment thereof, the anti-MASP2 antibody or antigen-binding fragment thereof comprising a heavy chain variable region (VH) and a light chain variable region (VL), the VH comprising HCDR1, HCDR2 and HCDR3 regions, and the VL comprising LCDR1, LCDR2 and LCDR3 regions, wherein:
(i) the HCDR1, HCDR2 and HCDR3 regions have sequences identical to the HCDR1, HCDR2 and HCDR3 of a VH as shown in any one of SEQ ID NOs: 1-2, or sequences having up to 5, 4, 3, 2 or 1 mutation(s) compared thereto, and the LCDR1, LCDR2 and LCDR3 regions have sequences identical to the LCDR1, LCDR2 and LCDR3 of a VL as shown in any one of SEQ ID NOs: 3-5, or sequences having up to 5, 4, 3, 2 or 1 mutation(s) compared thereto;
(ii) the HCDR1, HCDR2 and HCDR3 regions have sequences identical to the HCDR1, HCDR2 and HCDR3 of a VH as shown in any one of SEQ ID NOs: 6-8 and 48-50, or sequences having up to 5, 4, 3, 2 or 1 mutation(s) compared thereto, and the LCDR1, LCDR2 and LCDR3 regions have sequences identical to the LCDR1, LCDR2 and LCDR3 of a VL as shown in any one of SEQ ID NOs: 9-11 and 51-55, or sequences having up to 5, 4, 3, 2 or 1 mutation(s) compared thereto; or
(iii) the HCDR1, HCDR2 and HCDR3 regions have sequences identical to the HCDR1, HCDR2 and HCDR3 of a VH as shown in any one of SEQ ID NOs: 12-15 and 56-59, or sequences having up to 5, 4, 3, 2 or 1 mutation(s) compared thereto, and the LCDR1, LCDR2 and LCDR3 regions have sequences identical to the LCDR1, LCDR2 and LCDR3 of a VL as shown in any one of SEQ ID NOs: 16-18 and 60-63, or sequences having up to 5, 4, 3, 2 or 1 mutation(s) compared thereto. In some embodiments, the VH comprises HCDR1, HCDR2 and HCDR3 regions, and the VL comprises LCDR1, LCDR2 and LCDR3 regions, wherein:
   (i) the HCDR1, HCDR2 and HCDR3 regions have sequences identical to the HCDR1, HCDR2 and HCDR3 of the VH as shown in SEQ ID NO: 1, or sequences having up to 5, 4, 3, 2 or 1 mutation(s) compared thereto, and the LCDR1, LCDR2 and LCDR3 regions have sequences identical to the LCDR1, LCDR2 and LCDR3 of the VL as shown in SEQ ID NO: 3, or sequences having up to 5, 4, 3, 2 or 1 mutation(s) compared thereto;
   (ii) the HCDR1, HCDR2 and HCDR3 regions have sequences identical to the HCDR1, HCDR2 and HCDR3 of the VH as shown in SEQ ID NO: 8, or sequences having up to 5, 4, 3, 2 or 1 mutation(s) compared thereto, and the LCDR1, LCDR2 and LCDR3 regions have sequences identical to the LCDR1, LCDR2 and LCDR3 of the VL as shown in any one of SEQ ID NOs: 11 and 51-53, or sequences having up to 5, 4, 3, 2 or 1 mutation(s) compared thereto;
   (iii) the HCDR1, HCDR2 and HCDR3 regions have sequences identical to the HCDR1, HCDR2 and HCDR3 of the VH as shown in SEQ ID NO: 50, or sequences having up to 5, 4, 3, 2 or 1 mutation(s) compared thereto, and the LCDR1, LCDR2 and LCDR3 regions have sequences identical to the LCDR1, LCDR2 and LCDR3 of the VL as shown in SEQ ID NO: 51 or 55, or sequences having up to 5, 4, 3, 2 or 1 mutation(s) compared thereto; or
   (iv) the HCDR1, HCDR2 and HCDR3 regions have sequences identical to the HCDR1, HCDR2 and HCDR3 of the VH as shown in SEQ ID NO: 15, or sequences having up to 5, 4, 3, 2 or 1 mutation(s) compared thereto, and the LCDR1, LCDR2 and LCDR3 regions have sequences identical to the LCDR1, LCDR2 and LCDR3 of the VL as shown in SEQ ID NO: 18 or 63, or sequences having up to 5, 4, 3, 2 or 1 mutation(s) compared thereto.

The mutation is selected from insertions, deletions and/or substitutions that do not affect function, and the substitution is preferably a conservative amino acid substitution.

In some embodiments, the VH has HCDR1, HCDR2 and HCDR3 identical in sequence to those of the VH as shown in SEQ ID NO: 1, and the VL has LCDR1, LCDR2 and LCDR3 identical in sequence to those of the VL as shown in SEQ ID NO: 3 or 5; or, the VH has HCDR1, HCDR2 and HCDR3 identical in sequence to those of the VH as shown in SEQ ID NO: 2, and the VL has LCDR1, LCDR2 and LCDR3 identical in sequence to those of the VL as shown in SEQ ID NO: 4. In some embodiments, the VH has HCDR1, HCDR2 and HCDR3 identical in sequence to those of the VH as shown in SEQ ID NO: 6, and the VL has LCDR1, LCDR2 and LCDR3 identical in sequence to those of the VL as shown in SEQ ID NO: 9; the VH has HCDR1, HCDR2 and HCDR3 identical in sequence to those of the VH as shown in SEQ ID NO: 7, and the VL has LCDR1, LCDR2 and LCDR3 identical in sequence to those of the VL as shown in SEQ ID NO: 10 or 11; the VH has HCDR1, HCDR2 and HCDR3 identical in sequence to those of the VH as shown in SEQ ID NO: 8, and the VL has LCDR1, LCDR2 and LCDR3 identical in sequence to those of the VL as shown in any one of SEQ ID NOs: 11 and 51-53; the VH has HCDR1, HCDR2 and HCDR3 identical in sequence to those of the VH as shown in SEQ ID NO: 48, and the VL has LCDR1, LCDR2 and LCDR3 identical in sequence to those of the VL as shown in SEQ ID NO: 54; the VH has HCDR1, HCDR2 and HCDR3 identical in sequence to those of the VH as shown in SEQ ID NO: 49, and the VL has LCDR1, LCDR2 and LCDR3 identical in sequence to those of the VL as shown in SEQ ID NO: 52; or, the VH has HCDR1, HCDR2 and HCDR3 identical in sequence to those of the VH as shown in SEQ ID NO: 50, and the VL has LCDR1, LCDR2 and LCDR3 identical in sequence to those of the VL as shown in SEQ ID NO: 51 or 55.

In some embodiments, the VH has HCDR1, HCDR2 and HCDR3 identical in sequence to those of the VH as shown in SEQ ID NO: 12, and the VL has LCDR1, LCDR2 and LCDR3 identical in sequence to those of the VL as shown in SEQ ID NO: 16; the VH has HCDR1, HCDR2 and HCDR3 identical in sequence to those of the VH as shown in SEQ ID NO: 13, and the VL has LCDR1, LCDR2 and LCDR3 identical in sequence to those of the VL as shown in SEQ ID NO: 17; the VH has HCDR1, HCDR2 and HCDR3 identical in sequence to those of the VH as shown in SEQ ID NO: 14, and the VL has LCDR1, LCDR2 and LCDR3 identical in sequence to those of the VL as shown in SEQ ID NO: 18; the VH has HCDR1, HCDR2 and HCDR3 identical in sequence to those of the VH as shown in SEQ ID NO: 15, and the VL has LCDR1, LCDR2 and LCDR3 identical in sequence to those of the VL as shown in SEQ ID NO: 18 or 62; the VH has HCDR1, HCDR2 and HCDR3 identical in sequence to those of the VH as shown in SEQ ID NO: 56, and the VL has LCDR1, LCDR2 and LCDR3 identical in sequence to those of the VL as shown in SEQ ID NO: 60; the VH has HCDR1, HCDR2 and HCDR3 identical in sequence to those of the VH as shown in SEQ ID NO: 57, and the VL has LCDR1, LCDR2 and LCDR3 identical in sequence to those of the VL as shown in SEQ ID NO: 61; or, the VH has HCDR1, HCDR2 and HCDR3 identical in sequence to those of the VH as shown in any one of SEQ ID NOs: 15 and 57-59, and the VL has LCDR1, LCDR2 and LCDR3 identical in sequence to those of the VL as shown in SEQ ID NO: 63.

In some preferred embodiments, the VH and VL have HCDR1-3 and LCDR1-3 identical in sequence to those of VH and VL selected from the group consisting of:
(1) VH as shown in SEQ ID NO: 8 and VL as shown in SEQ ID NO: 51;
(2) VH as shown in SEQ ID NO: 8 and VL as shown in SEQ ID NO: 52;
(3) VH as shown in SEQ ID NO: 8 and VL as shown in SEQ ID NO: 53;
(4) VH as shown in SEQ ID NO: 50 and VL as shown in SEQ ID NO: 51;
(5) VH as shown in SEQ ID NO: 50 and VL as shown in SEQ ID NO: 55; and
(6) VH as shown in SEQ ID NO: 15 and VL as shown in SEQ ID NO: 63.

In some embodiments, the HCDR1, HCDR2 and HCDR3 of the VH, and the LCDR1, LCDR2 and LCDR3 of the VL are defined according to IMGT, Kabat, Chothia, AbM, Contact or any combination thereof. In some preferred embodiments, they are defined according to a combination of IMGT and Kabat; according to a combination of AbM and Kabat; or according to a combination of IMGT, Kabat, Chothia, AbM and Contact.

In some embodiments, the VH has an amino acid sequence having at least 85%, 90%, 91%, 92%, 93%, 94%, 95%, 96%, 97%, 98%, 99% or 100% identity to a sequence as shown in any one of SEQ ID NOs: 1-2, and/or the VL has an amino acid sequence having at least 85%, 90%, 91%, 92%, 93%, 94%, 95%, 96%, 97%, 98%, 99% or 100% identity to a sequence as shown in any one of SEQ ID NOs: 3-5.

In some embodiments, the VH has an amino acid sequence having at least 85%, 90%, 91%, 92%, 93%, 94%, 95%, 96%, 97%, 98%, 99% or 100% identity to a sequence as shown in any one of SEQ ID NOs: 6-8 and 48-50, and/or the VL has an amino acid sequence having at least 85%, 90%, 91%, 92%, 93%, 94%, 95%, 96%, 97%, 98%, 99% or 100% identity to a sequence as shown in any one of SEQ ID NOs: 9-11 and 51-55.

In some embodiments, the VH has an amino acid sequence having at least 85%, 90%, 91%, 92%, 93%, 94%, 95%, 96%, 97%, 98%, 99% or 100% identity to a sequence as shown in any one of SEQ ID NOs: 12-15 and 56-59, and/or the VL has an amino acid sequence having at least 85%, 90%, 91%, 92%, 93%, 94%, 95%, 96%, 97%, 98%, 99% or 100% identity to a sequence as shown in any one of SEQ ID NOs: 16-18 and 60-63.

In some embodiments, the VH comprises the sequence as shown in SEQ ID NO: 1, and the VL comprises the sequence as shown in any one of SEQ ID NOs: 3-5; or, the VH comprises the sequence as shown in SEQ ID NO: 2, and the VL comprises the sequence as shown in any one of SEQ ID NOs: 3-5.

In some embodiments, the VH comprises the sequence as shown in SEQ ID NO: 6, and the VL comprises the sequence as shown in any one of SEQ ID NOs: 9-11 and 51-55; the VH comprises the sequence as shown in SEQ ID NO: 7, and the VL comprises the sequence as shown in any one of SEQ ID NOs: 9-11 and 51-55; the VH comprises the sequence as shown in SEQ ID NO: 8, and the VL comprises the sequence as shown in any one of SEQ ID NOs: 9-11 and 51-55; the VH comprises the sequence as shown in SEQ ID NO: 48, and the VL comprises the sequence as shown in any one of SEQ ID NOs: 9-11 and 51-55; the VH comprises the sequence as shown in SEQ ID NO: 49, and the VL comprises the sequence as shown in any one of SEQ ID NOs: 9-11 and 51-55; or, the VH comprises the sequence as shown in SEQ ID NO: 50, and the VL comprises the sequence as shown in any one of SEQ ID NOs: 9-11 and 51-55.

In some embodiments, the VH comprises the sequence as shown in SEQ ID NO: 12, and the VL comprises the sequence as shown in any one of SEQ ID NOs: 16-18 and 60-63; the VH comprises the sequence as shown in SEQ ID NO: 13, and the VL comprises the sequence as shown in any one of SEQ ID NOs: 16-18 and 60-63; the VH comprises the sequence as shown in SEQ ID NO: 14, and the VL comprises the sequence as shown in any one of SEQ ID NOs: 16-18 and 60-63; the VH comprises the sequence as shown in SEQ ID NO: 15, and the VL comprises the sequence as shown in any one of SEQ ID NOs: 16-18 and 60-63; the VH comprises the sequence as shown in SEQ ID NO: 56, and the VL comprises the sequence as shown in any one of SEQ ID NOs: 16-18 and 60-63; the VH comprises the sequence as shown in SEQ ID NO: 57, and the VL comprises the sequence as shown in any one of SEQ ID NOs: 16-18 and 60-63; the VH comprises the sequence as shown in SEQ ID NO: 58, and the VL comprises the sequence as shown in any one of SEQ ID NOs: 16-18 and 60-63; or, the VH comprises the sequence as shown in SEQ ID NO: 59, and the VL comprises the sequence as shown in any one of SEQ ID NOs: 16-18 and 60-63.

In some preferred embodiments, the VH and VL are selected from the group consisting of: the VH and VL respectively comprise or are the sequences as shown in SEQ ID NOs: 1 and 3; the VH and VL respectively comprise or are the sequences as shown in SEQ ID NOs: 2 and 4; the VH and VL respectively comprise or are the sequences as shown in SEQ ID NOs: 1 and 5; the VH and VL respectively comprise or are the sequences as shown in SEQ ID NOs: 6 and 9; the VH and VL respectively comprise or are the sequences as shown in SEQ ID NOs: 7 and 10; the VH and VL respectively comprise or are the sequences as shown in SEQ ID NOs: 7 and 11; the VH and VL respectively comprise or are the sequences as shown in SEQ ID NOs: 8 and 11; the VH and VL respectively comprise or are the sequences as shown in SEQ ID NOs: 8 and 51; the VH and VL respectively comprise or are the sequences as shown in SEQ ID NOs: 8 and 52; the VH and VL respectively comprise or are the sequences as shown in SEQ ID NOs: 8 and 53; the VH and VL respectively comprise or are the sequences as shown in SEQ ID NOs: 48 and 54; the VH and VL respectively comprise or are the sequences as shown in SEQ ID NOs: 49 and 52; the VH and VL respectively comprise or are the sequences as shown in SEQ ID NOs: 50 and 51; the VH and VL respectively comprise or are the sequences as shown in SEQ ID NOs: 50 and 55; the VH and VL respectively comprise or are the sequences as shown in SEQ ID NOs: 12 and 16; the VH and VL respectively comprise or are the sequences as shown in SEQ ID NOs: 13 and 17; the VH and VL respectively comprise or are the sequences as shown in SEQ ID NOs: 14 and 18; the VH and VL respectively comprise or are the sequences as shown in SEQ ID NOs: 15 and 18; the VH and VL respectively comprise or are the sequences as shown in SEQ ID NOs: 56 and 60; the VH and VL respectively comprise or are the sequences as shown in SEQ ID NOs: 57 and 61; the VH and VL respectively comprise or are the sequences as shown in SEQ ID NOs: 15 and 62; the VH and VL respectively comprise or are the sequences as shown in SEQ ID NOs: 58 and 63; the VH and VL respectively comprise or are the sequences as shown in SEQ ID NOs: 15 and 63; the VH and VL respectively comprise or are the sequences as shown in SEQ ID NOs: 59 and 63; and, the VH and VL respectively comprise or are the sequences as shown in SEQ ID NOs: 57 and 63.

In some preferred embodiments, the VH and VL respectively comprise or are the sequences as shown in SEQ ID NOs: 1 and 3; the VH and VL respectively comprise or are the sequences as shown in SEQ ID NOs: 8 and 11; the VH and VL respectively comprise or are the sequences as shown in SEQ ID NOs: 8 and 51; the VH and VL respectively comprise or are the sequences as shown in SEQ ID NOs: 8 and 52; the VH and VL respectively comprise or are the sequences as shown in SEQ ID NOs: 8 and 53; the VH and VL respectively comprise or are the sequences as shown in SEQ ID NOs: 50 and 51; the VH and VL respectively comprise or are the sequences as shown in SEQ ID NOs: 50 and 55; the VH and VL respectively comprise or are the sequences as shown in SEQ ID NOs: 15 and 63; or, the VH and VL respectively comprise or are the sequences as shown in SEQ ID NOs: 15 and 18.

In some embodiments, the anti-MASP2 antibody provided in the present disclosure further comprises a heavy chain constant region and a light chain constant region. In some embodiments, the heavy chain constant region is selected from a human IgG1 constant region, a human IgG2 constant region, a human IgG3 constant region, a human IgG4 constant region or a variant thereof, and the light chain constant region is selected from a human κ chain constant region, a human λ chain constant region or a variant thereof. In some embodiments, the heavy chain constant region is selected from an IgG4 constant region variant with increased FcRn binding affinity. In some embodiments, the heavy chain constant region is selected from an IgG4 constant region having any one or more mutations of S228P (corresponding to amino acid residue on position 108 of SEQ ID NO: 19), M428L (corresponding to amino acid residue on position 308 of SEQ ID NO: 20), N434S (corresponding to amino acid residue on position 314 of SEQ ID NO: 20), M252Y (corresponding to amino acid residue on position 132 of SEQ ID NO: 21), S254T (corresponding to amino acid residue on position 134 of SEQ ID NO: 21), and T256E (corresponding to amino acid residue on position 136 of SEQ ID NO: 21), all mutations are numbered according to EU numbering. In some preferred embodiments, the heavy chain constant region comprises a sequence as shown in any one of SEQ ID NOs: 19-21 or an amino acid sequence having at least 85%, 90%, 91%, 92%, 93%, 94%, 95%, 96%, 97%, 98%, 99% or 100% identity thereto, and the light chain constant region comprises a sequence as shown in any one of SEQ ID NOs: 22-23 or an amino acid sequence having at least 85%, 90%, 91%, 92%, 93%, 94%, 95%, 96%, 97%, 98%, 99% or 100% identity thereto.

In some embodiments, the anti-MASP2 antibody provided in the present disclosure comprises a heavy chain and a light chain, the heavy chain has an amino acid sequence having at least 85%, 90%, 91%, 92%, 93%, 94%, 95%, 96%, 97%, 98%, 99% or 100% identity to a sequence as shown in any one of SEQ ID NOs: 37, 39, 42, and 43, and/or the light chain has an amino acid sequence having at least 85%, 90%, 91%, 92%, 93%, 94%, 95%, 96%, 97%, 98%, 99% or 100% identity to a sequence as shown in any one of SEQ ID NOs: 38, 40, and 41.

In some embodiments, the anti-MASP2 antibody provided in the present disclosure comprises a heavy chain and a light chain, the heavy chain has an amino acid sequence having at least 85%, 90%, 91%, 92%, 93%, 94%, 95%, 96%, 97%, 98%, 99% or 100% identity to a sequence as shown in any one of SEQ ID NOs: 24, 28, 31, 44, 67, 69, 70, 80, and 81, and/or the light chain has an amino acid sequence having at least 85%, 90%, 91%, 92%, 93%, 94%, 95%, 96%, 97%, 98%, 99% or 100% identity to a sequence as shown in any one of SEQ ID NOs: 25, 29, 30, 64, 65, 66, 68, and 71.

In some embodiments, the anti-MASP2 antibody provided in the present disclosure comprises a heavy chain and a light chain, the heavy chain has an amino acid sequence having at least 85%, 90%, 91%, 92%, 93%, 94%, 95%, 96%, 97%, 98%, 99% or 100% identity to a sequence as shown in any one of SEQ ID NOs: 26, 32, 34, 36, 45, 72, 74, 77, and 79, and/or the light chain has an amino acid sequence having at least 85%, 90%, 91%, 92%, 93%, 94%, 95%, 96%, 97%, 98%, 99% or 100% identity to a sequence as shown in any one of SEQ ID NOs: 27, 33, 35, 73, 75, 76, and 78.

In some embodiments, the heavy chain comprises the sequence as shown in SEQ ID NO: 37 and the light chain comprises the sequence as shown in any one of SEQ ID NOs: 38, 40, and 41; the heavy chain comprises the sequence as shown in SEQ ID NO: 39 and the light chain comprises the sequence as shown in any one of SEQ ID NOs: 38, 40, and 41; the heavy chain comprises the sequence as shown in SEQ ID NO: 42 and the light chain comprises the sequence as shown in any one of SEQ ID NOs: 38, 40, and 41; or, the heavy chain comprises the sequence as shown in SEQ ID NO: 43 and the light chain comprises the sequence as shown in any one of SEQ ID NOs: 38, 40, and 41.

In some embodiments, the heavy chain comprises the sequence as shown in SEQ ID NO: 24 and the light chain comprises the sequence as shown in any one of SEQ ID NOs: 25, 29, 30, 64, 65, 66, 68, and 71; the heavy chain comprises the sequence as shown in SEQ ID NO: 28 and the light chain comprises the sequence as shown in any one of SEQ ID NOs: 25, 29, 30, 64, 65, 66, 68, and 71; the heavy chain comprises the sequence as shown in SEQ ID NO: 31 and the light chain comprises the sequence as shown in any one of SEQ ID NOs: 25, 29, 30, 64, 65, 66, 68, and 71; the heavy chain comprises the sequence as shown in SEQ ID NO: 44 and the light chain comprises the sequence as shown in any one of SEQ ID NOs: 25, 29, 30, 64, 65, 66, 68, and 71; the heavy chain comprises the sequence as shown in SEQ ID NO: 67 and the light chain comprises the sequence as shown in any one of SEQ ID NOs: 25, 29, 30, 64, 65, 66, 68, and 71; the heavy chain comprises the sequence as shown in SEQ ID NO: 69 and the light chain comprises the sequence as shown in any one of SEQ ID NOs: 25, 29, 30, 64, 65, 66, 68, and 71; the heavy chain comprises the sequence as shown in SEQ ID NO: 70 and the light chain comprises the sequence as shown in any one of SEQ ID NOs: 25, 29, 30, 64, 65, 66, 68, and 71; the heavy chain comprises the sequence as shown in SEQ ID NO: 80 and the light chain comprises the sequence as shown in any one of SEQ ID NOs: 25, 29, 30, 64, 65, 66, 68, and 71; or, the heavy chain comprises the sequence as shown in SEQ ID NO: 81 and the light chain comprises the sequence as shown in any one of SEQ ID NOs: 25, 29, 30, 64, 65, 66, 68, and 71.

In some embodiments, the heavy chain comprises the sequence as shown in SEQ ID NO: 26 and the light chain comprises the sequence as shown in any one of SEQ ID NOs: 27, 33, 35, 73, 75, 76, and 78; the heavy chain comprises the sequence as shown in SEQ ID NO: 32 and the light chain comprises the sequence as shown in any one of SEQ ID NOs: 27, 33, 35, 73, 75, 76, and 78; the heavy chain comprises the sequence as shown in SEQ ID NO: 34 and the light chain comprises the sequence as shown in any one of SEQ ID NOs: 27, 33, 35, 73, 75, 76, and 78; the heavy chain comprises the sequence as shown in SEQ ID NO: 36 and the light chain comprises the sequence as shown in any one of SEQ ID NOs: 27, 33, 35, 73, 75, 76, and 78; the heavy chain comprises the sequence as shown in SEQ ID NO: 45 and the light chain comprises the sequence as shown in any one of SEQ ID NOs: 27, 33, 35, 73, 75, 76, and 78; the heavy chain comprises the sequence as shown in SEQ ID NO: 72 and the light chain comprises the sequence as shown in any one of SEQ ID NOs: 27, 33, 35, 73, 75, 76, and 78; the heavy chain comprises the sequence as shown in SEQ ID NO: 74 and the light chain comprises the sequence as shown in any one of SEQ ID NOs: 27, 33, 35, 73, 75, 76, and 78; the heavy chain comprises the sequence as shown in SEQ ID NO: 77 and the light chain comprises the sequence as shown in any one of SEQ ID NOs: 27, 33, 35, 73, 75, 76, and 78; or, the heavy chain comprises the sequence as shown in SEQ ID NO: 79 and the light chain comprises the sequence as shown in any one of SEQ ID NOs: 27, 33, 35, 73, 75, 76, and 78.

In some preferred embodiments, the heavy chain and light chain respectively comprise sequences set forth in a pair selected from the group consisting of: sequences as shown in SEQ ID NOs: 24 and 25; sequences as shown in SEQ ID NOs: 26 and 27; sequences as shown in SEQ ID NOs: 28 and 29; sequences as shown in SEQ ID NOs: 28 and 30; sequences as shown in SEQ ID NOs: 31 and 30; sequences as shown in SEQ ID NOs: 31 and 64; sequences as shown in SEQ ID NOs: 31 and 65; sequences as shown in SEQ ID NOs: 31 and 66; sequences as shown in SEQ ID NOs: 67 and 68; sequences as shown in SEQ ID NOs: 69 and 65; sequences as shown in SEQ ID NOs: 70 and 64; sequences as shown in SEQ ID NOs: 70 and 71; sequences as shown in SEQ ID NOs: 32 and 33; sequences as shown in SEQ ID NOs: 34 and 35; sequences as shown in SEQ ID NOs: 36 and 35; sequences as shown in SEQ ID NOs: 37 and 38; sequences as shown in SEQ ID NOs: 39 and 40; sequences as shown in SEQ ID NOs: 37 and 41; sequences as shown in SEQ ID NOs: 72 and 73; sequences as shown in SEQ ID NOs: 74 and 75; sequences as shown in SEQ ID NOs: 36 and 76; sequences as shown in SEQ ID NOs: 77 and 78; sequences as shown in SEQ ID NOs: 36 and 78; sequences as shown in SEQ ID NOs: 79 and 78; sequences as shown in SEQ ID NOs: 74 and 78; sequences as shown in SEQ ID NOs: 42 and 38; sequences as shown in SEQ ID NOs: 43 and 38; sequences as shown in SEQ ID NOs: 44 and 30; sequences as shown in SEQ ID NOs: 44 and 64; sequences as shown in SEQ ID NOs: 44 and 65; sequences as shown in SEQ ID NOs: 44 and 66; sequences as shown in SEQ ID NOs: 80 and 68; sequences as shown in SEQ ID NOs: 81 and 65; and, sequences as shown in SEQ ID NOs: 45 and 35.

In some preferred embodiments, the heavy chain and light chain respectively comprise sequences as shown in SEQ ID NOs: 31 and 30; the heavy chain and light chain respectively comprise sequences as shown in SEQ ID NOs: 31 and 64; the heavy chain and light chain respectively comprise sequences as shown in SEQ ID NOs: 31 and 65; the heavy chain and light chain respectively comprise sequences as shown in SEQ ID NOs: 31 and 66; the heavy chain and light chain respectively comprise sequences as shown in SEQ ID NOs: 70 and 64; the heavy chain and light chain respectively comprise sequences as shown in SEQ ID NOs: 70 and 71; the heavy chain and light chain respectively comprise sequences as shown in SEQ ID NOs: 36 and 78; the heavy chain and light chain respectively comprise sequences as shown in SEQ ID NOs: 36 and 35; the heavy chain and light chain respectively comprise sequences as shown in SEQ ID NOs: 37 and 38; the heavy chain and light chain respectively comprise sequences as shown in SEQ ID NOs: 42 and 38; the heavy chain and light chain respectively comprise sequences as shown in SEQ ID NOs: 43 and 38; the heavy chain and light chain respectively comprise sequences as shown in SEQ ID NOs: 44 and 30; the heavy chain and light chain respectively comprise sequences as shown in SEQ ID NOs: 44 and 64; the heavy chain and light chain respectively comprise sequences as shown in SEQ ID NOs: 44 and 65; the heavy chain and light chain respectively comprise sequences as shown in SEQ ID NOs: 44 and 66; or the heavy chain and light chain respectively comprise sequences as shown in SEQ ID NOs: 45 and 35.

In one aspect, the present disclosure provides an anti-MASP2 antibody or antigen-binding fragment thereof, which binds to or competes for binding to the same epitope as the aforementioned anti-MASP2 antibody or antigen-binding fragment thereof.

In some embodiments, the anti-MASP2 antibody or antigen-binding fragment thereof provided in the present disclosure has at least one of the following characteristics:
(1) the antibody or antigen-binding fragment thereof binds to human MASP2 with a KD of 10 nM or lower, the KD can be detected, for example, using the method of Example 3 of the present disclosure;
(2) the antibody or antigen-binding fragment thereof inhibits complement lectin pathway MAC deposition in an *in vitro* assay with an IC₅₀ of less than 1, less than 0.9, less than 0.8, less than 0.7, less than 0.6, less than 0.5, less than 0.4, less than 0.3, less than 0.2, less than 0.1, less than 0.09, less than 0.08, less than 0.07, less than 0.06, less than 0.05, less than 0.04, less than 0.03, less than 0.02, less than 0.01 µg/mL or lower, the IC₅₀ can be detected, for example, using the method of Example 4 of the present disclosure;
(3) the antibody or antigen-binding fragment thereof substantially does not inhibit the classical pathway and/or the alternative pathway;
(4) the antibody or antigen-binding fragment thereof substantially does not bind to complement components C1r, C1s, MASP1, and/or MASP3;
(5) when binding to human MASP2, the KD of the antibody or antigen-binding fragment thereof is substantially the same as or better than that of a reference MASP2 antibody;
(6) when binding to human MASP2, the antibody or antigen-binding fragment thereof specifically inhibits or blocks the complement lectin pathway, preferably, the inhibition or blocking effect of the antibody or antigen-binding fragment thereof is substantially the same as or better than that of a reference MASP2 antibody;
(7) when binding to human MASP2, the antibody or antigen-binding fragment thereof reduces or decreases C4b and/or MAC deposition, preferably, the antibody or antigen-binding fragment thereof reduces or decreases C4b and/or MAC deposition at a level substantially the same as or better than that of a reference MASP2 antibody.

In some preferred embodiments, the reference MASP2 antibody is selected from Narsoplimab.

In some embodiments, the anti-MASP2 antibody provided in the present disclosure is a chimeric antibody, a humanized antibody or a fully human antibody. In some embodiments, the anti-MASP2 antibody is a monoclonal antibody.

In some embodiments, the antigen-binding fragment of the present disclosure is selected from: Fab, F(ab')₂, Fab', Fd, Fv, dsFv, scFv, Fab, and diabody.

In some embodiments, the anti-MASP2 antibody or antigen-binding fragment thereof provided in the present disclosure comprises one or more effector molecules, the effector molecule is selected from an anti-tumor agent, a drug, a toxin, a bioactive protein (e.g., an enzyme), another antibody or antibody fragment, a synthetic or naturally occurring polymer, a nucleic acid and fragment thereof such as DNA, RNA and fragments thereof, a radionuclide (e.g., radioiodide), a radioisotope, a chelated metal, a nanoparticle and a reporter group (e.g., a fluorescent compound), and a compound detectable by NMR or ESR spectroscopy. In some specific embodiments, the effector molecule is conjugated or fused to the anti-MASP2 antibody or antigen-binding fragment thereof of the present disclosure.

In one aspect, the present disclosure provides a bispecific or multispecific antibody or antigen-binding fragment thereof, comprising the anti-MASP2 antibody or antigen-binding fragment thereof of the present disclosure.

In one aspect, the present disclosure provides a polynucleotide encoding the anti-MASP2 antibody or antigen-binding fragment thereof of the present disclosure. The polynucleotide of the present disclosure can be, for example, DNA or RNA, and may or may not contain intron sequences. In a preferred embodiment, the polynucleotide is a cDNA molecule. The polynucleotide of the present disclosure can be prepared or obtained by known methods based on the information of the amino acid sequence of the anti-MASP2 antibody or antigen-binding fragment thereof of the present disclosure, for example, by automatic DNA synthesis and/or recombinant DNA technology.

As is well known in the art, multiple codons can encode the same amino acid. Therefore, nucleic acids encoding a protein sequence include nucleic acids with codon degeneracy. The amino acid sequences described in the present disclosure can be encoded by a variety of nucleic acids. The genetic code is universal and well known. Nucleic acids encoding any of the amino acid sequences described in the present disclosure can be easily conceived based on common general knowledge in the art and can be optimized for production. Although the number of possible nucleic acid sequences encoding a given amino acid is large, given the standard table of the genetic code, and with the aid of a computer, those skilled in the art can readily generate every possible combination of nucleic acid sequences encoding a given amino acid.

In one aspect, the present disclosure provides an expression vector comprising the polynucleotide of the present disclosure. The expression vector includes an eukaryotic expression vector, a prokaryotic expression vector, a viral vector, such as a bacterial plasmid, a phage, a yeast plasmid, a plant cell virus, a mammalian cell virus such as an adenovirus, a retrovirus, or other vectors.

In one aspect, the present disclosure provides a host cell comprising the polynucleotide or expression vector of the present disclosure. The host cell comprises a prokaryotic cell, a fungal cell or a mammalian cell, such as a CHO cell, an NS0 cell or other mammalian cells, *E. coli* or other prokaryotic cells, a yeast cell or other fungal cells.

In one aspect, the present disclosure provides a pharmaceutical composition comprising the anti-MASP2 antibody or antigen-binding fragment thereof, or the bispecific or multispecific antibody or antigen-binding fragment thereof, or the polynucleotide, or the expression vector, or the host cell of the present disclosure, and one or more pharmaceutically acceptable carriers.

In some preferred embodiments, the pharmaceutical composition contains a therapeutically effective amount of the aforementioned anti-MASP2 antibody or antigen-binding fragment thereof, or the aforementioned nucleic acid molecule, and one or more pharmaceutically acceptable carriers, diluents, buffers or excipients.

In some embodiments, the pharmaceutical composition may contain any number of excipients.

Excipients that can be used include carriers, surfactants, thickening or emulsifying agents, solid binders, dispersion or suspension aids, solubilizers, coloring agents, flavoring agents, coating agents, disintegrants, lubricants, sweeteners, preservatives, isotonic agents, or combinations thereof. The selection and use of suitable excipients are taught in Gennaro, ed., Remington: The Science and Practice of Pharmacy, 20th Ed. (Lippincott Williams & Wilkins 2003), the disclosure of which is incorporated herein by reference.

In some embodiments, the pharmaceutical composition further comprises one or more other therapeutic agents.

In one aspect, the present disclosure provides a kit comprising the aforementioned anti-MASP2 antibody or antigen-binding fragment thereof.

In one aspect, the present disclosure provides use of the aforementioned anti-MASP2 antibody or antigen-binding fragment thereof in detecting MASP2 protein or preparing a reagent for detecting MASP2 protein. MASP2 protein can be detected *in vivo* or *in vitro*, preferably human MASP2 protein, which can be for non-disease diagnostic purposes.

In one aspect, the present disclosure provides a method for preventing and/or treating a disease or disorder associated with MASP2 in a subject, comprising administering a therapeutically or prophylactically effective amount of the anti-MASP2 antibody or antigen-binding fragment thereof, the bispecific or multispecific antibody or antigen-binding fragment thereof, the polynucleotide, the expression vector, the host cell, the pharmaceutical composition or the kit of the present disclosure to a subject in need thereof.

In one aspect, the present disclosure provides use of the aforementioned anti-MASP2 antibody or antigen-binding fragment thereof, or the aforementioned bispecific or multispecific antibody or antigen-binding fragment thereof, or the aforementioned polynucleotide, or the aforementioned expression vector, or the aforementioned host cell, or the aforementioned pharmaceutical composition, or the aforementioned kit in the preparation of a medicament for preventing and/or treating a disease or disorder associated with MASP2 in a subject.

In some embodiments, the disease or disorder associated with MASP2 is a disease or disorder caused by abnormal expression of MASP2. In some embodiments, the disease associated with MASP2 is selected from a disease or disorder caused by overexpression of MASP2. In some preferred embodiments, the disease associated with MASP2 is selected from IgA nephropathy, thrombotic microangiopathy, systemic lupus erythematosus, membranous nephropathy, lupus nephritis, transplant rejection, antiphospholipid syndrome, rheumatoid arthritis, hemolytic uremic syndrome, hereditary angioedema, myocardial infarction, etc.

In one aspect, the present disclosure provides use of the aforementioned anti-MASP2 antibody or antigen-binding fragment thereof, or the aforementioned bispecific or multispecific antibody or antigen-binding fragment thereof, or the aforementioned polynucleotide, or the aforementioned expression vector, or the aforementioned host cell, or the aforementioned pharmaceutical composition, or the aforementioned kit in the preparation of a medicament, wherein the medicament is used for:
(1) specifically inhibiting or blocking the complement lectin pathway; and/or
(2) reducing or decreasing C4b and/or MAC deposition.

The present disclosure also provides a method for preparing the anti-MASP2 antibody or antigen-binding fragment thereof of the present disclosure:
The anti-MASP2 antibody or antigen-binding fragment thereof described in the present disclosure can be obtained using conventional techniques, such as in the *Antibodies: A Laboratory Manual*, Cold Spring Harbor, Chapters 5-8 and 15. For example, mice can be immunized with human MASP2 or fragments thereof, the resulting antibodies can be renatured, purified, and subjected to amino acid sequencing using conventional methods. Antigen-binding fragments can also be prepared using conventional methods. The antibody or antigen-binding fragment described in the present disclosure is prepared by genetic engineering methods adding one or more human FR regions to non-human CDR regions. Human FR germline sequences can be obtained by aligning the IMGT human antibody variable region germline gene database and MOE software from the ImMunoGeneTics (IMGT) website, or from the Immunoglobulin Journal, 2001 ISBN012441351. Alternatively, for example, the cDNA sequences encoding the heavy and light chains are cloned into an expression vector. The recombinant immunoglobulin expression vector can be stably transfected into CHO cells. Stable clones are obtained by expressing antibodies that specifically bind to human MASP2. Positive clones are expanded in serum-free medium in a bioreactor to produce antibodies.

The anti-MASP2 antibody or antigen-binding fragment thereof of the present disclosure can be expressed intracellularly, or on the cell membrane, or secreted extracellularly. If necessary, the recombinant protein can be isolated and purified using various separation methods based on its physical, chemical and other properties. These methods are well known to those skilled in the art. Generally, the transformed host cells are cultured under conditions suitable for expression of the antibody of the present disclosure, and then the anti-MASP2 antibody or antigen-binding fragment thereof of the present disclosure is purified using conventional immunoglobulin purification steps, such as Protein A-Sepharose affinity chromatography, ion exchange chromatography, hydrophobic chromatography, molecular sieve chromatography, hydroxyapatite chromatography, gel electrophoresis, dialysis and other conventional separation and purification means and combinations thereof. The antibody can be filtrated and concentrated by using conventional methods.

As a preferred embodiment of the method for preparing the anti-MASP2 antibody or antigen-binding fragment thereof according to the present disclosure, the method for isolating and purifying the anti-MASP2 antibody or antigen-binding fragment thereof is Protein A affinity chromatography, cation exchange or anion exchange.

The obtained monoclonal antibody or bispecific antibody can be identified by conventional means. For example, the binding specificity of the antibody can be determined by immunoprecipitation or *in vitro* binding assays, such as enzyme-linked immunosorbent assay (ELISA) or radioimmunoassay (RIA). The binding affinity of the antibody can be determined, for example, by Scatchard analysis of Munson et al., Anal. Biochem., 107:220 (1980), or by surface plasmon resonance (Biacore).

For clarity, general terms used in the description of compounds are defined herein.

Unless otherwise stated, the following terms and phrases used herein are intended to have the following meanings. A specific term or phrase should not be considered uncertain or unclear without a special definition, but should be understood according to its ordinary meaning. When a trade name appears herein, it is intended to refer to its corresponding product or its active ingredient. The term "pharmaceutically acceptable" as used herein refers to those compounds, materials, compositions, and/or dosage forms which are, within the scope of sound medical judgment, suitable for use in contact with the tissues of humans and animals without excessive toxicity, irritation, allergic response, or other problems or complications, commensurate with a reasonable benefit/risk ratio.

The terms "mannan-binding lectin-associated serine protease 2", "MASP2 protein", "MASP2 antigen" and "MASP2" are used interchangeably and mean any MASP2 molecule and functional homologs thereof known to those skilled in the art. Unless otherwise indicated, MASP2 includes but is not limited to those derived from humans, rodents, mice, rats, primates, monkeys and guinea pigs. The term also refers to fragments or variants of natural MASP2 that retain at least one *in vivo* or *in vitro* activity of natural MASP2. The term includes the full-length unprocessed precursor form of MASP2 as well as the mature form resulting from post-translational cleavage of the signal peptide. MASP2 as used herein also refers to specific polypeptides expressed in cells via naturally occurring DNA sequence variations of the MASP2 gene, such as single nucleotide polymorphisms of the MASP2 gene. Examples include the amino acid sequence of human MASP2 registered under Genbank accession number NP_006601.2, the amino acid sequence of mouse MASP2 registered under Genbank accession number NP_001003893.1, the amino acid sequence of rat MASP2 registered under Genbank accession number NP_742040.1, the amino acid sequence of monkey MASP2 registered under Genbank accession number XP_045236890.1 or XP_005544871.1. Other examples of amino acid sequences of MASP2 can be obtained using, for example, GenBank, UniProt, and OMIM.

The term "antibody" as used herein includes intact antibodies and any antigen-binding fragments thereof (i.e., "antigen-binding portions") or single chains thereof. An intact antibody is a glycoprotein comprising two heavy (H) chains and two light (L) chains interconnected by disulfide bonds. Each heavy chain is composed of a heavy chain variable region (abbreviated herein as VH) and a heavy chain constant region (abbreviated herein as CH). The heavy chain constant region is composed of three domains, CH1, CH2 and CH3. Each light chain is composed of a light chain variable region (abbreviated herein as VL) and a light chain constant region (abbreviated herein as CL). The light chain constant region is composed of one domain, CL. The VH and VL regions can be further subdivided into hypervariable regions (termed complementarity determining regions (CDRs)), interspersed with more conserved regions, termed framework regions (FRs). Each VH and VL is composed of three CDRs and four FRs, arranged from amino-terminus to carboxy-terminus in the following order: FR1, CDR1, FR2, CDR2, FR3, CDR3, and FR4. The three CDRs of the light chain are referred to as LCDR1, LCDR2, and LCDR3; the three CDRs of the heavy chain are referred to as HCDR1, HCDR2 and HCDR3. The variable regions of the heavy and light chains contain a binding domain that interacts with the antigen. The constant region of the antibody can mediate the binding of the immunoglobulin to host tissues or factors, wherein the host tissues or factors include various cells of the immune system (e.g., effector cells) and the first component (C1q) of the classical complement system.

For the determination or definition of CDRs, the definitive delineation of CDRs and the identification of residues comprising the binding site of an antibody can be accomplished by resolving the structure of the antibody and/or resolving the structure of the antibody-ligand complex. This can be achieved by any of various techniques known to those skilled in the art, such as X-ray crystallography. Various analytical methods (including combinations of methods) can be used to identify CDRs, including but not limited to Kabat, Chothia, AbM, IMGT, and Contact definitions, and conformational definitions. All of these are well known techniques in the art, see, e.g., Kabat, E.A. et al. (1991) Sequences of Proteins of Immunological Interest, Fifth Edition, U.S. Department of Health and Human Services, NIH Publication No. 91-3242; Chothia et al. (1989) Nature 342:877; Chothia, C. et al. (1987) J. Mol. Biol. 196:901-917; Al-lazikani et al. (1997) J. Molec. Biol. 273:927-948; and Almagro, J. Mol. Recognit. 17:132-143 (2004). Software programs used include but are not limited to AbRSA (http://cao.labshare.cn/AbRSA/cdrs.php), abYsis (www.abysis.org/abysis/sequence_input/key_annotation/key_annotation.cgi) and IMGT (http://www.imgt.org/3Dstructure-DB/cgi/DomainGapAlign.cgi#results). The positions of amino acid residues comprised by an exemplary definition of CDRs are listed in the table below:

| **CDR Loop** | **Kabat** | **AbM** | **Chothia¹** | **Contact²** | **IMGT** |
|---|---|---|---|---|---|
| LCDR1 | L24--L34 | L24--L34 | L24--L34 | L30--L36 | L27--L32 |
| LCDR2 | L50--L56 | L50--L56 | L50--L56 | L46--L55 | L50--L51 |
| LCDR3 | L89--L97 | L89--L97 | L89--L97 | L89--L96 | L89--L97 |
| HCDR1 | H31--H35B (Kabat Numbering)³ | H26--H35B | H26--H32..34 | H30--H35B | H26--H35B |
| HCDR1 | H31--H35 (Chothia Numbering) | H26--H35 | H26--H32 | H30--H35 | H26--H33 |
| HCDR2 | H50--H65 | H50--H58 | H52--H56 | H47--H58 | H51--H56 |
| HCDR3 | H95--H102 | H95--H102 | H95--H102 | H93--H101 | H93--H102 |

| | | | | | |
|---|---|---|---|---|---|
| Note 1: Definitions vary slightly in different literatures, especially the Chothia definition scheme; Note 2: Except for the Contact definition which uses Chothia numbering or Martin numbering scheme, other definition schemes are compatible with various numbering schemes; Note 3: When using Kabat numbering, the end of Chothia HCDR1 varies between H32 and H34 depending on the length of the loop (because the Kabat numbering scheme places insertions at H35A and H35B). Note: Amino acid numbers on the heavy chain are indicated by "H + number", and amino acid numbers on the light chain are indicated by "L + number"; for example, L24-L34 in the second row and second column of the table refers to the amino acid sequence determined from position 24 to position 34 according to the Kabat numbering scheme from the N-terminus of the antibody light chain variable region; and so on. | | | | | |

The term "antigen-binding fragment" (or simply "antibody portion") refers to one or more fragments of an antibody that specifically bind to an antigen (e.g., MASP2 protein). It has been shown that the antigen-binding function of an antibody can be performed by fragments of a full-length antibody. Examples of binding fragments encompassed by the term "antigen-binding fragment" of an antibody include: (i) a Fab fragment, a monovalent fragment consisting of the VL, VH, CL and CH1 domains; (ii) an F(ab')₂ fragment, a bivalent fragment comprising two Fab fragments linked by a disulfide bridge at the hinge region; (iii) an Fd fragment consisting of the VH and CH1 domains; (iv) an Fv fragment consisting of the VL and VH domains of a single arm of an antibody; (v) a dAb fragment consisting of a VH domain (Ward et al., (1989) Nature 341:544-546); (vi) an isolated complementarity determining region (CDR); and (vii) a nanobody, a heavy chain variable region comprising a single variable domain and two constant domains. Furthermore, although the two domains of the Fv fragment, VL and VH, are encoded by separate genes, they can be joined, using recombinant methods, by a linker that enables them to be made as a single protein chain in which the VL and VH regions pair to form monovalent molecules (known as single chain Fv (scFv); see, e.g., Bird et al., (1988) Science 242:423-426; and Huston et al., (1988) Proc. Natl. Acad. Sci. USA 85:5879-5883). Such single chain antibodies are also encompassed within the term "antigen-binding fragment" of an antibody. These antibody fragments can be obtained by conventional techniques known to those skilled in the art, and the fragments are screened for use in the same manner as intact antibodies.

As used herein, the term "monoclonal antibody" refers to a formulation of antibody molecules composed of single molecular. A monoclonal antibody composition exhibits a single binding specificity and affinity for a particular epitope.

The term "murine antibody" refers to a monoclonal antibody prepared against human MASP2 according to knowledge and skill in the art. During preparation, a test subject can be injected with a MASP2 antigen, and then a hybridoma expressing an antibody with a desired sequence or functional characteristic is isolated. The murine anti-MASP2 antibody or antigen-binding fragment thereof may further comprise a light chain constant region of murine κ chain, murine λ chain or a variant thereof, or further comprise a heavy chain constant region of murine IgG1, murine IgG2, murine IgG3 or a variant thereof.

The term "chimeric antibody" is an antibody formed by fusing the variable region of a heterologous (e.g., murine) antibody with the constant region of a parent antibody (e.g., a human antibody), which can reduce the immune response elicited by the heterologous antibody. For example, for a human-mouse chimeric antibody, to establish a chimeric antibody, a hybridoma secreting murine specific monoclonal antibody is first established, then the variable region gene is cloned from the murine hybridoma cell, then the constant region gene of the human antibody is cloned as needed, the murine variable region gene is linked to the human constant region gene to form a chimeric gene which is then inserted into an expression vector, and finally the chimeric antibody molecule is expressed in a eukaryotic or prokaryotic system.

The term "humanized antibody", also known as CDR-grafted antibody, refers to an antibody produced by grafting murine CDR sequences into a human antibody variable region framework, i.e., different types of human germline antibody framework sequences. This can overcome the heterologous reaction induced by chimeric antibodies due to the large amount of murine protein components they carry. Such framework sequences can be obtained from public DNA databases including germline antibody gene sequences or from published references. For example, germline DNA sequences of human heavy and light chain variable region genes can be obtained from the "VBase" human germline sequence database, and also found in Kabat, E.A. et al., 1991 Sequences of Proteins of Immunological Interest, 5th Edition. To avoid decreased activity while reducing immunogenicity, the human antibody variable region framework sequence may be subjected to minimal back mutation or reversion mutation to maintain activity.

The term "bispecific" or "multispecific" means that the antibody and/or antigen-binding molecule is capable of specifically binding two or more different antigenic determinants, typically, a bispecific antibody or antigen-binding molecule comprises two antigen-binding sites, each specific for a different antigenic determinant. In certain embodiments, the bispecific or multispecific antibody or antigen-binding molecule is capable of binding two or more antigenic determinants simultaneously, particularly two or more antigenic determinants expressed on two or more different cells.

The term "variant" of a heavy chain constant region and a light chain constant region refers to a variant of heavy chain constant regions or light chain constant regions derived from human known in the art that does not alter the structure and function of the antibody variable region. Exemplary variants include IgG1, IgG2, IgG3 or IgG4 heavy chain constant region variants with site-directed modification and amino acid substitution in the heavy chain constant region, specific substitutions known in the art, such as YTE mutation, L234A and/or L235A mutation, S228P mutation, and/or mutations that introduce a knob-into-hole structure (such that the antibody heavy chains form a combination of knob-Fc and hole-Fc), all of which have been demonstrated to confer novel characteristics to the antibody but do not alter the function of the antibody variable region.

As used herein, the terms "comprise", "include/contain" and "have" are used interchangeably and are intended to have an inclusive meaning, meaning that the solution may include other elements in addition to those listed. It should also be understood that where "comprise", "include/contain" and "have" are used herein, they also provide for the solution "consisting of...".

The term "antibody" herein includes, but is not limited to, monoclonal antibodies, polyclonal antibodies, monospecific antibodies, multispecific antibodies (e.g., bispecific antibodies), monovalent antibodies, multivalent antibodies, intact antibodies, antigen-binding fragments, naked antibodies, conjugated antibodies, humanized antibodies or fully human antibodies.

The term "conservative amino acid" herein generally refers to amino acids belonging to the same class or sharing similar characteristics (e.g., charge, side-chain size, hydrophobicity, hydrophilicity, backbone conformation, and rigidity). Exemplarily, amino acids within each of the following groups are conservative amino acid residues with respect to each other, and substitution of amino acid residues within a group is a conservative amino acid substitution:
1) Alanine (A), Serine (S), Threonine (T);
2) Aspartic acid (D), Glutamic acid (E);
3) Asparagine (N), Glutamine (Q);
4) Arginine (R), Lysine (K), Histidine (H);
5) Isoleucine (I), Leucine (L), Methionine (M), Valine (V); and
6) Phenylalanine (F), Tyrosine (Y), Tryptophan (W).

The terms "identity" and "sequence identity" are used interchangeably herein and are calculated by: To determine the percent identity of two amino acid sequences or two nucleic acid sequences, the sequences are aligned for optimal comparison purposes (e.g., gaps can be introduced in one or both of the first and second amino acid sequences or nucleic acid sequences for optimal alignment, or non-homologous sequences can be discarded for comparison purposes). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. When the term "competition/compete" is used in the context of antigen-binding proteins (e.g., neutralizing antigen-binding proteins or neutralizing antibodies) that compete for the same epitope, it means competition between antigen-binding proteins as determined by an assay in which the antigen-binding protein to be tested (e.g., an antibody or immunologically functional fragment thereof) prevents or inhibits (e.g., reduces) the specific binding of a reference antigen-binding protein (e.g., a ligand or a reference antibody) to a common antigen (e.g., a MASP2 antigen or fragment thereof). Numerous types of competitive binding assays can be used to determine whether one antigen-binding protein competes with another, such assays are for example: solid phase direct or indirect radioimmunoassay (RIA), solid phase direct or indirect enzyme immunoassay (EIA), sandwich competition assay (see, e.g., Stahli et al., 1983, Methods in Enzymology 9:242-253); solid phase direct biotin-avidin EIA (see, e.g., Kirkland et al., 1986, J. Immunol. 137:3614-3619), solid phase direct labeled assay, solid phase direct labeled sandwich assay (see, e.g., Harlow and Lane, 1988, Antibodies, A Laboratory Manual, Cold Spring Harbor Press); solid phase direct labeled RIA with I-125 label (see, e.g., Morel et al., 1988, Molec. Immunol. 25:7-15); solid phase direct biotin-avidin EIA (see, e.g., Cheung, et al., 1990, Virology 176:546-552); and directly labeled RIA (Moldenhauer et al., 1990, Scand. J. Immunol. 32:77-82). Typically, the assay involves the use of purified antigen bound to a solid surface or cells bearing either unlabeled test antigen-binding protein and labeled reference antigen-binding protein. Competitive inhibition is measured by determining the amount of label bound to the solid surface or cells in the presence of the test antigen-binding protein. Usually, the test antigen-binding protein is present in excess. Antigen-binding proteins identified by competitive assay (competing antigen-binding proteins) include: antigen-binding proteins that bind to the same epitope as the reference antigen-binding protein; and antigen-binding proteins that bind to an adjacent epitope sufficiently close to the binding epitope of the reference antigen-binding protein such that the two epitopes sterically interfere with binding.

Typically, when the competing antigen-binding protein is present in excess, it will inhibit (e.g., reduce) specific binding of the reference antigen-binding protein to the common antigen by at least 40-45%, 45-50%, 50-55%, 55-60%, 60-65%, 65-70%, 70-75% or 75% or more. In certain cases, binding is inhibited by at least 80-85%, 85-90%, 90-95%, 95-97% or 97% or more.

The term "subject" includes any human or non-human animal. The term "non-human animal" includes all vertebrates, e.g., mammals and non-mammals, such as non-human primates, rodents, rabbits, pigs, dogs, cats, chickens, amphibians, and reptiles, though mammals such as non-human primates and rodents are preferred.

The term "therapeutically effective amount" refers to an amount of the anti-MASP2 antibody or antigen-binding fragment thereof of the present disclosure sufficient to prevent or ameliorate symptoms associated with a disease or condition (e.g., IgA nephropathy), and/or to reduce the severity of the disease or condition. A therapeutically effective amount should be understood in the context of the condition being treated, where those skilled in the art can readily recognize the actual effective amount.

The antibodies of the present disclosure are monoclonal antibodies characterized structurally and chemically as described below and in the following examples. The amino acid sequence ID numbers of the heavy chain/light chain variable regions of exemplary antibodies are summarized in Table 1, and the heavy chain and light chain constant region sequence ID numbers are summarized in Table 2.

**Table 1. Heavy chain/light chain variable region sequences of anti-MASP2 antibodies of the present disclosure**

| | | |
|---|---|---|
| 20W series antibodies | VH1 | EVQLVESGGGVVQPGRSLRLSCAASGFTFPSYAMHWVRQAPGKGLEWVAVISYDLSNKYYA DSVKGRFTISRDNSKNTLYLQMNSLRΛEDTΛVYYCΛKSRRVYYYYGMDVWGQGTTVTVSS (SEQ ID NO: 1) |
| | VH2 | EVQLVESGGGVVQPGRSLRLSCAASGFTFPSYAMYWVRQAPGKGLEWVAVISYDLSNKLYA DSVKGRFTISRDNSKNTLYLQMNSLRAEDTAVYYCAKSRRQYYYYGMDVWGQGTTVTVSS (SEQ ID NO: 2) |
| | VL1 | SYVLTQPPSVSVSPGQTASITCSAGKLGDKFASWYQQKPGQSPVLVIYQDSKRGSGIPERFSGS NSGNTATLTISGTQAMDEADYYCQAWDSSGYVFGTGTKVTVL (SEQ ID NO: 3) |
| | VL2 | SYVLTQPPSVSVSPGQTASITCSGGKLGDKFASWYQQKPGQSPVLVIYQDSKRGRGIPERFSGS NSGNTATLTISGTQAMDEADYYCQAWDSSTYVFGTGTKVTVL (SEQ ID NO: 4) |
| | VL3 | SYVLTQPPSVSVSPGQTASITCSGYKLGDKFASWYQQKPGQSPVLVIYQDSKRGSGIPERFSGS NSGNTATLTISGTQAMDEADYYCQAWDSSGYVFGTGTKVTVL (SEQ ID NO: 5) |
| 11W series antibodies | VH1 | EVQLQQSGAEVVKPGASVKLSCKASGYTFTSYWIHWVKQRPGQGLEWIGELNPSNGRTNYN EKFKSKATLTVDKSSNTAYMQVSSLTSEDSAVYYCARWAYDAMDYWGQGTSVTVSS (SEQ ID NO: 6) |
| | VH2 | QVQLVQSGAEVKKPGASVKVSCKASGYTFTSYWIHWVRQAPGQGLEWMGELNPSNGRTNY NEKFKSRVTMTVDKSTNTAYMELSSLRSEDTAVYYCARWAYDAMDYWGQGTLVTVSS (SEQ ID NO: 7) |
| | VH3 | QVQLVQSGAEVKKPGASVKLSCKASGYTFTSYWIHWVRQAPGQGLEWMGELNPSNGRTNY NEKFKSRVTLTRDKSTSTAYMQVSSLRSEDTAVYYCARWAYDAMDYWGQGTLVTVSS (SEQ ID NO: 8) |
| | VH4 | QVQLVQSGAEVKKPGASVKLSCKASGYTFTSYGIHWVRQAPGQGLEWMGELNPSNGRTNY NEKFKSRVTLTRDKSTSTAYMQVSSLRSEDTAVYYCARWAYDALDYWGQGTLVTVSS (SEQ ID NO: 48) |
| | VH5 | QVQLVQSGAEVKKPGASVKLSCKASGYTFTSYGIHWVRQAPGQGLEWMGELNPSNGRTNY NEKFKSRVTLTRDKSTSTAYMQVSSLRSEDTAVYYCARWAYDAMDYWGQGTLVTVSS (SEQ ID NO: 49) |
| | VH6 | EVQLVQSGAEVKKPGASVKLSCKASGYTFTSYWIHWVRQAPGQGLEWMGELNPSNGRTNY NEKFKSRVTLTVDKSTSTAYMEVSSLTSEDTAVYYCARWAYDAMDYWGQGTLVTVSS (SEQ ID NO: 50) |
| | VL1 | DIVLTQSPASLAVSLGQRATISCRASESVDSYGNSFMHWYQQKPGQPPKLLIYLASNLESGVP ARFSGSGSRTDFTLTIDPVEADDAATYYCQQNNEDPWTFGGGTKLEIK (SEQ ID NO: 9) |
| | VL2 | DIVLTQSPASLAVSPGQRATITCRASESVDSYGNSFMHWYQQKPGQPPKLLIYLASNLESGVP ARFSGSGSRTDFTLTINPVEADDTATYYCQQNNEDPWTFGQGTKVEIK (SEQ ID NO: 10) |
| | VL3 | DIVLTQSPASLAVSPGQRATITCRASESVDSYGNSFMHWYQQKPGQPPKLLIYLASNLESGVP ARFSGSGSRTDFTLTIDPVEADDAANYYCQQNNEDPWTFGQGTKVEIK (SEQ ID NO: 11) |
| | VL4 | DIVLTQSPASLAVSPGQRATITCRASESVDSYGNSFMHWYQQKPGQPPKLLIYYASNLESGVP ARFSGSGSRTDFTLTIDPVEADDAANYYCQQNNEDPWTFGQGTKVEIK (SEQ ID NO: 51) |
| | VL5 | DIVLTQSPASLAVSPGQRATITCRASESVDSYGNSFMHWYQQKPGQPPKLLIYHASNLESGVP ARFSGSGSRTDFTLTIDPVEADDAANYYCQQNNEDPWTFGQGTKVEIK (SEQ ID NO: 52) |
| | VL6 | DIVLTQSPASLAVSPGQRATITCRASESVDSYGASFMHWYQQKPGQPPKLLIYYASNLESGVP ARFSGSGSRTDFTLTIDPVEADDAANYYCQQNNEDPWTFGQGTKVEIK (SEQ ID NO: 53) |
| | VL7 | DIVLTQSPASLAVSPGQRATITCRASESVDSYGASFMHWYQQKPGQPPKLLIYHASNLESGVP ARFSGSGSRTDFTLTIDPVEADDAANYYCQQNNEDPWTFGQGTKVEIK (SEQ ID NO: 54) |
| | VL8 | DIVLTQSPASLAVSPGQRATITCRASESVDSYGNSFMHWYQQKPGQPPKLLIYYASNLESGVP ARFSGSGSRTDFTLTINPVEADDAANYYCQQNNEDPWTFGQGTKVEIK (SEQ ID NO: 55) |
| 12W series antibodies | VH1 | QVQLQQSGPELVKPGASVKISCKASGYAFSNSWMNWVKQRPGKGLEWIGRIYPGDGDINYN GKFKGKATLTADKSSSIAYMQLSSLTSEDSAVYFCARGGRDEGSMDYWGQGASVTVSS (SEQ ID NO: 12) |
| | VH2 | QVQLVQSGAEVKKPGASVKVSCKASGYAFSNSWMNWVRQAPGQGLEWMGRIYPGDGDINY NGKFKGRVTMTRDTSISTAYMELSSLRSDDTAVYYCARGGRDEGSMDYWGQGTLVTVSS (SEQ ID NO: 13) |
| | VH3 | QVQLVQSGAEVKKPGASVKVSCKASGYAFSNSWMNWVRQAPGQGLEWMGRIYPGDGDINY NGKFKGRVTMTRDTSSSIAYMELSSLRSDDTAVYYCARGGRDEGSMDYWGQGTLVTVSS (SEQ ID NO: 14) |
| | VH4 | QVQLVQSGAEVKKPGASVKVSCKASGYAFSNSWMNWVRQAPGKGLEWMGRIYPGDGDINY NGKFKGRVTMTADTSSSTAYMELSSLRSDDTAVYYCARGGRDEGSMDYWGQGTLVTVSS (SEQ ID NO: 15) |
| | VH5 | QVQLVQSGAEVKKPGASVKVSCKASGYAFSNSWMNWVRQAPGKGLEWMGRIYPGDGDINY NGKFKGRVTMTADTSSSTAYMELSSLRSDDTAVYYCARGGRDEGSHDYWGQGTLVTVSS (SEQ ID NO: 56) |
| | VH6 | QVQLVQSGAEVKKPGASVKVSCKASGYAFSNSWMDWVRQAPGKGLEWMGRIYPEDGDINY NGKFKGRVTMTADTSSSTAYMELSSLRSDDTAVYYCARGGRDEGSMDYWGQGTLVTVSS (SEQ ID NO: 57) |
| | VH7 | QVQLVQSGAEVKKPGASVKVSCKASGYAFSNSWMDWVRQAPGKGLEWMGRIYPGDGDINY NGKFKGRVTMTADTSSSTAYMELSSLRSDDTAVYYCARGGRDEGSMDYWGQGTLVTVSS (SEQ ID NO: 58) |
| | VH8 | QVQLVQSGAEVKKPGASVKVSCKASGYAFSNSWMNWVRQAPGKGLEWMGRIYPEDGDINY NGKFKGRVTMTADTSSSTAYMELSSLRSDDTAVYYCARGGRDEGSMDYWGQGTLVTVSS (SEQ ID NO: 59) |
| | VL1 | DIVMTQSQKFMSTSVGDRVSVTCKASQNVGINVAWYQQKPGQSPKPLIYSASYRYSGVPDRF TGSGSGTDFTLTISNVQSEDLAEYFCQQYYSHFLFTFGSGTKLEIK (SEQ ID NO: 16) |
| | VL2 | DIQMTQSPSSLSASVGDRVTITCKASQNVGINVAWYQQKPGKAPKLLIYSASYRYSGVPDRFS GSGSGTDFTLTISNLQSEDFATYFCQQYYSHFLFTFGQGTKVEIK (SEQ ID NO: 17) |
| | VL3 | DIVMTQSPSSLSASVGDRVTVTCKASQNVGINVAWYQQKPGQAPKPLIYSASYRYSGVPDRFS GSGSGTDFTLTISSLQSEDFAEYYCQQYYSHFLFTFGQGTKVEIK (SEQ ID NO: 18) |
| | VL4 | DIVMTQSPSSLSASVGDRVTVTCKASQNVGINVAWYQQKPGQAPKPLIYSASYRYSGVPDRFS GSGSGTDFTLTISSLQSEDFAEYYCKQYYSHFLFSFGQGTKVEIK (SEQ ID NO: 60) |
| | VL5 | DIVMTQSPSSLSASVGDRVTVTCKASQNVGINVAWYQQKPGQAPKPLIYSASYRESGVPDRFS GSGSGTDFTLTISSLQSEDFAEYYCQQYYSHFLFTFGQGTKVEIK (SEQ ID NO: 61) |
| | VL6 | DIVMTQSPSSLSASVGDRVTVTCKASQNRGINVAWYQQKPGQAPKPLIYSASYRESGVPDRFS GSGSGTDFTLTISSLQSEDFAEYYCQQYYSHFLFTFGQGTKVEIK (SEQ ID NO: 62) |
| | VL7 | DIVMTQSPSSLSASVGDRVTVTCKASQNRGINVAWYQQKPGQAPKPLIYSASYRESGVPDRFS GSGSGTDFTLTISSLQSEDFAEYYCQQYYSHRLFTFGQGTKVEIK (SEQ ID NO: 63) |

| | | |
|---|---|---|
| Note: The underlined amino acid residues are the CDR regions defined by Kabat. | | |

**Table 2. Heavy chain/light chain constant region sequences of anti-MASP2 antibodies of the present disclosure**

| | | |
|---|---|---|
| Heavy chain constant region | IgG4SP | ASTKGPSVFPLAPCSRSTSESTAALGCLVKDYFPEPVTVSWNSGALTSGVHTFPAVLQSSGLYSL SSVVTVPSSSLGTKTYTCNVDHKPSNTKVDKRVESKYGPPCPPCPAPEFLGGPSVFLFPPKPKDT LMISRTPEVTCVVVDVSQEDPEVQFNWYVDGVEVHNAKTKPREEQFNSTYRVVSVLTVLHQD WLNGKEYKCKVSNKGLPSSIEKTISKAKGQPREPQVYTLPPSQEEMTKNQVSLTCLVKGFYPSD IAVEWESNGQPENNYKTTPPVLDSDGSFFLYSRLTVDKSRWQEGNVFSCSVMHEALHNHYTQK SLSLSLGK (SEQ ID NO: 19) |
| | IgG4SP-LS | ASTKGPSVFPLAPCSRSTSESTAALGCLVKDYFPEPVTVSWNSGALTSGVHTFPAVLQSSGLYSL SSVVTVPSSSLGTKTYTCNVDHKPSNTKVDKRVESKYGPPCPPCPAPEFLGGPSVFLFPPKPKDT LMISRTPEVTCVVVDVSQEDPEVQFNWYVDGVEVHNAKTKPREEQFNSTYRVVSVLTVLHQD WLNGKEYKCKVSNKGLPSSIEKTISKAKGQPREPQVYTLPPSQEEMTKNQVSLTCLVKGFYPSD IAVEWESNGQPENNYKTTPPVLDSDGSFFLYSRLTVDKSRWQEGNVFSCSVLHEALHSHYTQKS LSLSLGK (SEQ ID NO: 20) |
| | IgG4SP-YTE | ASTKGPSVFPLAPCSRSTSESTAALGCLVKDYFPEPVTVSWNSGALTSGVHTFPAVLQSSGLYSL SSVVTVPSSSLGTKTYTCNVDHKPSNTKVDKRVESKYGPPCPPCPAPEFLGGPSVFLFPPKPKDT LYITREPEVTCVVVDVSQEDPEVQFNWYVDGVEVHNAKTKPREEQFNSTYRVVSVLTVLHQD WLNGKEYKCKVSNKGLPSSIEKTISKAKGQPREPQVYTLPPSQEEMTKNQVSLTCLVKGFYPSD IAVEWESNGQPENNYKTTPPVLDSDGSFFLYSRLTVDKSRWQEGNVFSCSVMHEALHNHYTQK SLSLSLGK(SEQ ID NO: 21) |
| Light chain constant region | λ chain | GQPKAAPSVTLFPPSSEELQANKATLVCLISDFYPGAVTVAWKADSSPVKAGVETTTPSKQSNN KYAASSYLSLTPEQWKSHRSYSCQVTHEGSTVEKTVAPTECS (SEQ ID NO: 22) |
| | κ chain |  (SEQ ID NO: 23) |

The positive control antibody Narsoplimab used in the present disclosure was synthesized according to WO2012151481A, and its heavy chain/light chain amino acid sequences are as follows:
>Narsoplimab heavy chain (SEQ ID NO: 46): 
QVTLKESGPVLVKPTETLTLTCTVSGFSLSRGKMGVSWIRQPPGKALEWLAHIFSSDEKS
YRTSLKSRLTISKDTSKNQVVLTMTNMDPVDTATYYCARIRRGGIDYWGQGTLVTVSSA
STKGPSVFPLAPCSRSTSESTAALGCLVKDYFPEPVTVSWNSGALTSGVHTFPAVLQSSG
LYSLSSVVTVPSSSLGTKTYTCNVDHKPSNTKVDKRVESKYGPPCPPCPAPEFLGGPSVFL
FPPKPKDTLMISRTPEVTCVVVDVSQEDPEVQFNWYVDGVEVHNAKTKPREEQFNSTYR
VVSVLTVLHQDWLNGKEYKCKVSNKGLPSSIEKTISKAKGQPREPQVYTLPPSQEEMTK
NQVSLTCLVKGFYPSDIAVEWESNGQPENNYKTTPPVLDSDGSFFLYSRLTVDKSRWQE
GNVFSCSVMHEALHNHYTQKSLSLSLGK.
>Narsoplimab light chain (SEQ ID NO: 47):

QPVLTQPPSLSVSPGQTASITCSGEKLGDKYAYWYQQKPGQSPVLVMYQDKQRPSGIPE
RFSGSNSGNTATLTISGTQAMDEADYYCQAWDSSTAVFGGGTKLTVLGQPKAAPSVTLF
PPSSEELQANKATLVCLISDFYPGAVTVAWKADSSPVKAGVETTTPSKQSNNKYAASSY
LSLTPEQWKSHRSYSCQVTHEGSTVEKTVAPTECS.

The CDR sequences of the heavy chain variable regions and light chain variable regions in Table 1 as defined by Kabat, Chothia, IMGT, AbM, and Contact using abYsis are detailed in Table 3.

**Table 3. CDR sequences of anti-MASP2 antibodies of the present disclosure**

| Antibody ID | Numbering System/ Method | HCDR | Amino acid sequence | LCDR | Amino acid sequence |
|---|---|---|---|---|---|
| 20W series antibo dies | Chothia | HCDR1 | GFTFPSY | LCDR1 | SX₁X₂KLGDKFAS, wherein X₁ is A or G, and X₂ is G or Y |
| | | HCDR2 | SYDLSN | LCDR2 | QDSKRGX₁, wherein X₁ is S or R |
| | | HCDR3 | SRRX₁YYYYGMDV, wherein X₁ is V or Q | LCDR3 | QAWDSSX₁ YV, wherein X₁ is G or T |
| | AbM | HCDR1 | GFTFPSYAMX₁, wherein X₁ is H or Y | LCDR1 | SX₁X₂KLGDKFAS, wherein X₁ is A or G, and X₂ is G or Y |
| | | HCDR2 | VISYDLSNKX₁, wherein X₁ is Y or L | LCDR2 | QDSKRGX₁, wherein X₁ is S or R |
| | | HCDR3 | SRRX₁YYYYGMDV, wherein X₁ is V or Q | LCDR3 | QAWDSSX₁ YV, wherein X₁ is G or T |
| | Kabat | HCDR1 | SYAMX₁, wherein X₁ is H or Y | LCDR1 | SX₁X₂KLGDKFAS, wherein X₁ is A or G, and X₂ is G or Y |
| | | HCDR2 | VISYDLSNKX₁ YADSVKG, wherein X₁ is Y or L | LCDR2 | QDSKRGX₁, wherein X₁ is S or R |
| | | HCDR3 | SRRX₁YYYYGMDV, wherein X₁ is V or Q | LCDR3 | QAWDSSX₁ YV, wherein X₁ is G or T |
| | Contact | HCDR1 | PSYAMX₁, wherein X₁ is H or Y | LCDR1 | DKFASWY |
| | | HCDR2 | WVAVISYDLSNKX₁, wherein X₁ is Y or L | LCDR2 | LVIYQDSKRG |
| | | HCDR3 | AKSRRX₁ YYYYGMD, wherein X₁ is V or Q | LCDR3 | QAWDSSX₁ Y, wherein X₁ is G or T |
| | IMGT | HCDR1 | GFTFPSYA | LCDR1 | KLGDKF |
| | | HCDR2 | ISYDLSNK | LCDR2 | QD |
| | | HCDR3 | AKSRRX₁ YYYYGMDV, wherein X₁ is V or Q | LCDR3 | QAWDSSX₁ YV, wherein X₁ is G or T |
| 11W series antibo dies | Chothia | HCDR1 | GYTFTSY | LCDR1 | RASESVDSYGX₁ SFMH, wherein X₁ is N or A |
| | | HCDR2 | NPSNGR | LCDR2 | X₁ASNLES, wherein X₁ is L, H or Y |
| | | HCDR3 | WAYDAX₁DY, wherein X₁ is M or L | LCDR3 | QQNNEDPWT |
| | AbM | HCDR1 | GYTFTSYX₁IH, wherein X₁ is W or G | LCDR1 | RASESVDSYGX₁ SFMH, wherein X₁ is N or A |
| | | HCDR2 | ELNPSNGRTN | LCDR2 | X₁ASNLES, wherein X₁ is L, H or Y |
| | | HCDR3 | WAYDAX₁DY, wherein X₁ is M or L | LCDR3 | QQNNEDPWT |
| | Kabat | HCDR1 | SYX₁IH, wherein X₁ is W or G | LCDR1 | RASESVDSYGX₁ SFMH, wherein X₁ is N or A |
| | | HCDR2 | ELNPSNGRTNYNEKFKS | LCDR2 | X₁ASNLES, wherein X₁ is L, H or Y |
| | | HCDR3 | WAYDAX₁DY, wherein X₁ is M or L | LCDR3 | QQNNEDPWT |
| | Contact | HCDR1 | TSYX₁IH, wherein X₁ is W or G | LCDR1 | DSYGX₁SFMHWY, wherein X₁ is N or A |
| | | HCDR2 | WX₁GELNPSNGRTN, wherein X₁ is I or M | LCDR2 | LLIYX₁ASNLE, wherein X₁ is L, H or Y |
| | | HCDR3 | ARWAYDAX₁D, wherein X₁ is M or L | LCDR3 | QQNNEDPW |
| | IMGT | HCDR1 | GYTFTSYX₁, wherein X₁ is W or G | LCDR1 | ESVDSYGX₁SF, wherein X₁ is N or A |
| | | HCDR2 | LNPSNGRT | LCDR2 | X₁A, wherein X₁ is L, H or Y |
| | | HCDR3 | ARWAYDAX₁DY, wherein X₁ is M or L | LCDR3 | QQNNEDPWT |
| 12W series antibo dies | Chothia | HCDR1 | GYAFSNS | LCDR1 | KASQNX₁GINVA, wherein X₁ is V or R |
| | | HCDR2 | YPX₁DGD, wherein X₁ is G or E | LCDR2 | SASYRX₁S, wherein X1 is Y or E |
| | | HCDR3 | GGRDEGSX₁DY, wherein X₁ is M or H | LCDR3 | X₁QYYSHX₂LFX₃, wherein X₁ is Q or K, X₂ is F or R, and X₃ is T or S |
| | AbM | HCDR1 | GYAFSNSWMX₁, wherein X₁ is N or D | LCDR1 | KASQNX₁GINVA, wherein X₁ is V or R |
| | | HCDR2 | RIYPX₁DGDIN, wherein X₁ is G or E | LCDR2 | SASYRX₁S, wherein X1 is Y or E |
| | | HCDR3 | GGRDEGSX₁DY, wherein X₁ is M or H | LCDR3 | X₁QYYSHX₂LFX₃, wherein X₁ is Q or K, X₂ is F or R, and X₃ is T or S |
| | Kabat | HCDR1 | NSWMX₁, wherein X₁ is N or D | LCDR1 | KASQNX₁GINVA, wherein X₁ is V or R |
| | | HCDR2 | RIYPX₁DGDINYNGKFKG, wherein X₁ is G or E | LCDR2 | SASYRX₁S, wherein X₁ is Y or E |
| | | HCDR3 | GGRDEGSX₁DY, wherein X₁ is M or H | LCDR3 | X₁QYYSHX₂LFX₃, wherein X₁ is Q or K, X₂ is F or R, and X₃ is T or S |
| | Contact | HCDR1 | SNSWMX₁, wherein X₁ is N or D | LCDR1 | GINVAWY |
| | | HCDR2 | WX₁GRIYPX₂DGDIN, wherein X₁ is I or M, and X₂ is G or E | LCDR2 | X₁LIYSASYRX₂, wherein X₁ is P or L, and X₂ is Y or E |
| | | HCDR3 | ARGGRDEGSX₁D, wherein X₁ is M or H | LCDR3 | X₁QYYSHX₂LF, wherein X₁ is Q or K, and X₂ is F or R |
| | IMGT | HCDR1 | GYAFSNSW | LCDR1 | QNX₁GIN, wherein X₁ is V or R |
| | | HCDR2 | IYPX₁DGDI, wherein X₁ is G or E | LCDR2 | SA |
| | | HCDR3 | ARGGRDEGSX₁DY, wherein X₁ is M or H | LCDR3 | X₁QYYSHX₂LFX₃, wherein X₁ is Q or K, X₂ is F or R, and X₃ is T or S |

Other features and advantages of the present disclosure will become apparent from the following detailed description and examples, which should not be construed as limiting. The contents of all references, Genbank entries, patents, and published patent applications cited throughout this application are expressly incorporated herein by reference.

### BRIEF DESCRIPTION OF THE DRAWINGS

Figure 1 shows the effect of antibodies 11W0 and 11W3 on the complement classical pathway, wherein an isotype IgG antibody (IgG4SP) serves as a negative control.
Figure 2 shows the effect of antibodies 11W0 and 11W3 on the alternative pathway, wherein an isotype IgG antibody (IgG4SP) serves as a negative control.
Figure 3 shows the binding of antibodies 11W0, 11W1, and 11W3 to the complement family members, with Narsoplimab as a control antibody. Among them, the complement family member in Figure 3A is human complement component C1s, the complement family member in Figure 3B is human complement component C1r, the complement family member in Figure 3C is human complement component MASP-1, and the complement family member in Figure 3D is human complement component MASP-3.
Figure 4 shows the inhibitory activity of antibodies 20W30, 11W3 and their long-acting modified antibodies on the complement lectin pathway *in vitro*.
Figure 5A shows the inhibitory activity of the long-acting modified antibody 11W4-LS on the complement lectin pathway *in vitro* (human complement serum), with Narsoplimab as a control antibody; the IC₅₀ value of 11W4-LS is <0.01 µg/mL.
Figure 5B shows the inhibitory activity of the long-acting modified antibody 11W4-LS on the complement lectin pathway *in vitro* (cynomolgus monkey complement serum), with Narsoplimab as a control antibody.
Figure 5C shows the inhibitory activity of long-acting modified antibodies 11W5-LS, 11W6-LS, 11W7-LS, and 11W8-LS on the complement lectin pathway *in vitro* (human complement serum), with Narsoplimab as a control antibody; the IC₅₀ values of 11W5-LS, 11W6-LS, 11W7-LS, and 11W8-LS are <0.05 µg/mL.
Figure 6 shows the affinity assay results of some antibodies by ELISA.
Figure 7 shows the results of complement lectin pathway MAC assay for some antibodies (human complement serum).
Figure 8 shows the effect of some antibodies on the complement classical pathway and alternative pathway (human complement serum).
Figure 9 shows the effect of some antibodies on C4b deposition (human complement serum).

### DETAILED DESCRIPTION OF THE INVENTION

### Example 1. Preparation of Anti-MASP2 Antibodies by Mouse Immunization

### 1.1 Preparation of murine antibodies

A total of 12 Balb/C mice (Vital River Laboratories) and 12 C57 mice (Vital River Laboratories) were immunized by injection of protein, and the proteins used were huMASP-2A-chis, cynoMASP-2A-chis, musMASP-2A-chis, and ratMASP-2A-chis. Freund's complete adjuvant was used for the first immunization (100 µg/mouse), and Freund's incomplete adjuvant was used for the second, third, and fourth immunizations (50 µg/mouse). A booster immunization was performed with 100 µg/mouse. On day 3 after the booster immunization, mouse spleens and retro-orbital blood were collected for titer determination, and the spleens were homogenized and stored in Trizol.

Total RNA was extracted from the spleens of mice after the booster immunization using the Trizol method, and the extracted total RNA was reverse-transcribed into cDNA. Based on the germline of Fab heavy and light chains, the variable region genes of heavy and light chains in mice were amplified, and then cloned into a phage display vector by restriction enzyme digestion and ligation. The recombinant vector was transformed into competent *E. coli* SS320 cells (Lucigen, MC1061 F) to construct an antibody gene phage display library with a library capacity of 5×10⁸.

Library screening was performed by magnetic bead method and immunotube method. The magnetic bead method screening was based on the biotinylation of the antigen protein hu10AA-CCP1-CCP2-SP-A-chis, and then binding it to streptavidin-coupled magnetic beads. The panning process was performed by incubating the antigen-bound magnetic beads with the antibody gene phage display library, followed by washing and elution. Typically, after three rounds of panning, specific monoclonal antibodies against the antigen were enriched. The immunotube method screening was based on coating the antigen proteins hu10AA-CCP1-CCP2-SP-A-chis, cyno10AA-CCP1-CCP2-SP-A-chis2, mus10AA-CCP1-CCP2-SP-A-chis, and rat10AA-CCP1-CCP2-SP-A-chis onto the surface of immunotubes with high adsorption capacity. The panning process was performed by adding the phage display antibody library to the immunotubes and incubating with the antigen proteins adsorbed on the immunotube surface, followed by washing and elution. After three rounds of panning, specific monoclonal antibody Fabs against the antigen were enriched. ELISA was performed on the phage pool eluted from each round to evaluate the enrichment efficiency, and the third round was used for monoclonal selection. The monoclonal phage supernatant was collected after centrifugation and used for ELISA as follows: 1) Coating: 2 µg/mL of antigens huMASP-2A-chis, cynoMASP-2A-chis, ratMASP-2A-chis, and musMASP-2A-chis were coated, 30 µL/well, overnight at 4°C, and washed 3 times with PBST; 2) Blocking: the wells were blocked with 5% PBSM for 1 h at room temperature, and washed 3 times with PBST; 3) Primary antibody: monoclonal phage expression supernatant was added, a negative control (IgG) was diluted to 5 µg/mL, added at 30 µL/well, after incubation for 1 h at room temperature, the wells were washed 3 times with PBST; 4) Secondary antibody: secondary antibody anti-M13-HRP (1:20000 dilution in 5% PBSM) was added, and for the negative control (IgG) sample, secondary antibody anti-human-Fab-HRP (1:5000 dilution in PBS) was added, 30 µL/well, after incubation for 1 h at room temperature, the wells were washed 6 times with PBST; 5) Termination: TMB was added at 30 µL/well, and incubated for color development for 5 min-10 min at room temperature, followed by the addition of 30 µL/well of 2 M stop solution to terminate the reaction. The absorbance at OD₄₅₀ was measured using a microplate reader. For the positive clones identified, plasmids were extracted for next-generation sequencing. The amino acid sequences of the heavy chain variable regions and light chain variable regions of the murine anti-MASP2 antibodies obtained in this example are shown in Table 4.

**Table 4. Heavy chain/light chain variable region sequences of murine anti-MASP2 antibodies**

| | | |
|---|---|---|
| 11W | VH1 | EVQLQQSGAEVVKPGASVKLSCKASGYTFTSYWIHWVKQRPGQGLEWIGELNPSN GRTNYNEKFKSKATLTVDKSSNTAYMQVSSLTSEDSAVYYCARWAYDAMDYWG QGTSVTVSS (SEQ ID NO: 6) |
| | VL1 | DIVLTQSPASLAVSLGQRATISCRASESVDSYGNSFMHWYQQKPGQPPKLLIYLASN LESGVPARFSGSGSRTDFTLTIDPVEADDAATYYCQQNNEDPWTFGGGTKLEIK (SEQ ID NO: 9) |
| 12W | VH1 | QVQLQQSGPELVKPGASVKISCKASGYAFSNSWMNWVKQRPGKGLEWIGRIYPGD GDINYNGKFKGKATLTADKSSSIAYMQLSSLTSEDSAVYFCARGGRDEGSMDYWG QGASVTVSS (SEQ ID NO: 12) |
| | VL1 | DIVMTQSQKFMSTSVGDRVSVTCKASQNVGINVAWYQQKPGQSPKPLIYSASYRY SGVPDRFTGSGSGTDFTLTISNVQSEDLAEYFCQQYYSHFLFTFGSGTKLEIK (SEQ ID NO: 16) |

| | | |
|---|---|---|
| Note: The underlined amino acid residues are the CDR regions defined by the Kabat numbering system/method. | | |

### 1.2 Preparation of chimeric antibodies

The heavy chain variable region sequence of the murine anti-MASP2 antibody was cloned into a GSV0 vector containing a human heavy chain constant region (IgG4SP with S228P Fc modification to stabilize the hinge), the amino acid sequence of the heavy chain constant region is shown in SEQ ID NO: 19; the light chain variable region sequence of the murine anti-MASP2 antibody was cloned into a GSV0 vector containing a human light chain constant region (κ chain), the light chain constant region sequence is shown in SEQ ID NO: 23. Using the ExpiCHO expression system, the above vectors were co-transfected into CHO cells for expression, and the expression supernatant was collected and purified to obtain complete chimeric antibodies. The chimeric antibodies targeting MASP2, 11WO (11W-VH1-VL1) and 12WO (12W-VH1-VL1), have heavy and light chain sequences as follows:
>11WO heavy chain (SEQ ID NO: 24): 
EVQLQQSGAEVVKPGASVKLSCKASGYTFTSYWIHWVKQRPGQGLEWIGELNPSNGRT
NYNEKFKSKATLTVDKSSNTAYMQVSSLTSEDSAVYYCARWAYDAMDYWGQGTSVT
VSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVMHEALHNHYTQKSLSLSLGK.
>11WO light chain (SEQ ID NO: 25): 
DIVLTQSPASLAVSLGQRATISCRASESVDSYGNSFMHWYQQKPGQPPKLLIYLASNLES
GVPARFSGSGSRTDFTLTIDPVEADDAATYYCQQNNEDPWTFGGGTKLEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC.
>12WO heavy chain (SEQ ID NO: 26): 
QVQLQQSGPELVKPGASVKISCKASGYAFSNSWMNWVKQRPGKGLEWIGRIYPGDGDI
NYNGKFKGKATLTADKSSSIAYMQLSSLTSEDSAVYFCARGGRDEGSMDYWGQGASVT
VSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVMHEALHNHYTQKSLSLSLGK.
>12WO light chain (SEQ ID NO: 27):

DIVMTQSQKFMSTSVGDRVSVTCKASQNVGINVAWYQQKPGQSPKPLIYSASYRYSGVP
DRFTGSGSGTDFTLTISNVQSEDLAEYFCQQYYSHFLFTFGSGTKLEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC.

### 1.3 Preparation of humanized antibodies

The structure of the parent antibody was modeled using the MOE (Molecular Operating Environment) homology modeling program, and then CDR grafting was used to design humanized antibodies. Briefly, the CDRs of the parent antibody were grafted into human antibody frameworks to obtain humanized light and heavy chains for each parent antibody. Furthermore, based on the humanized sequences, post-translational modification (PTM) sites, such as glycosylation sites, deamidation sites, isomerization sites, etc., were identified, and mutations were designed to remove PTM sites to optimize the antibody sequence, thereby avoiding negative impacts on protein conformation and function.

Depending on the degree of humanization substitution, multiple humanized sequences can be generated simultaneously based on the parent sequences. Exemplary humanized heavy chain variable region and light chain variable region sequences are shown in Table 1, which shows 5 humanized VHs (i.e., 11W-VH2 to 11W-VH6) designed for 11W-VH1, and 7 humanized VLs (i.e., 11W-VL2 to 11W-VL8) designed for 11W-VL1; 7 humanized VHs (i.e., 12W-VH2 to 12W-VH8) designed for 12W-VH1, and 6 humanized VLs (i.e., 12W-VL2 to 12W-VL7) designed for 12W-VL1.

The heavy chain constant region of the antibody can be selected from a human IgG1 constant region, a human IgG2 constant region, a human IgG3 constant region, a human IgG4 constant region or variants thereof, and the light chain constant region can be selected from the light chain constant region of human κ chain, human λ chain or variants thereof. The heavy chain/light chain constant regions shown in Table 2 were combined with the heavy chain/light chain variable regions shown in Table 1 to form complete anti-MASP2 antibodies. Exemplary humanized antibody heavy chain/light chain sequences are as follows:
>11W1 heavy chain (SEQ ID NO: 28): 
QVQLVQSGAEVKKPGASVKVSCKASGYTFTSYWIHWVRQAPGQGLEWMGELNPSNGR
TNYNEKFKSRVTMTVDKSTNTAYMELSSLRSEDTAVYYCARWAYDAMDYWGQGTLV
TVSSASTKGPSVFPLAPCSRSTSESTAALGCLVKDYFPEPVTVSWNSGALTSGVHTFPAV
LQSSGLYSLSSVVTVPSSSLGTKTYTCNVDHKPSNTKVDKRVESKYGPPCPPCPAPEFLG
GPSVFLFPPKPKDTLMISRTPEVTCVVVDVSQEDPEVQFNWYVDGVEVHNAKTKPREEQ
FNSTYRVVSVLTVLHQDWLNGKEYKCKVSNKGLPSSIEKTISKAKGQPREPQVYTLPPSQ
EEMTKNQVSLTCLVKGFYPSDIAVEWESNGQPENNYKTTPPVLDSDGSFFLYSRLTVDK
SRWQEGNVFSCSVMHEALHNHYTQKSLSLSLGK.
>11W1 light chain (SEQ ID NO: 29): 
DIVLTQSPASLAVSPGQRATITCRASESVDSYGNSFMHWYQQKPGQPPKLLIYLASNLES
GVPARFSGSGSRTDFTLTINPVEADDTATYYCQQNNEDPWTFGQGTKVEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC.
>11W2 heavy chain: SEQ ID NO: 28
>11W2 light chain (SEQ ID NO: 30): 
DIVLTQSPASLAVSPGQRATITCRASESVDSYGNSFMHWYQQKPGQPPKLLIYLASNLES
GVPARFSGSGSRTDFTLTIDPVEADDAANYYCQQNNEDPWTFGQGTKVEIKRTVAAPSV
 
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC.
>11W3 heavy chain (SEQ ID NO: 31): 
QVQLVQSGAEVKKPGASVKLSCKASGYTFTSYWIHWVRQAPGQGLEWMGELNPSNGR
TNYNEKFKSRVTLTRDKSTSTAYMQVSSLRSEDTAVYYCARWAYDAMDYWGQGTLVT
VSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLIIQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVMHEALHNHYTQKSLSLSLGK.
>11W3 light chain: SEQ ID NO: 30
>11W4 heavy chain (SEQ ID NO: 31): 
QVQLVQSGAEVKKPGASVKLSCKASGYTFTSYWIHWVRQAPGQGLEWMGELNPSNGR
TNYNEKFKSRVTLTRDKSTSTAYMQVSSLRSEDTAVYYCARWAYDAMDYWGQGTLVT
VSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLIIQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVMHEALHNHYTQKSLSLSLGK.
>11W4 light chain (SEQ ID NO: 64): 
DIVLTQSPASLAVSPGQRATITCRASESVDSYGNSFMHWYQQKPGQPPKLLIYYASNLES
GVPARFSGSGSRTDFTLTIDPVEADDAANYYCQQNNEDPWTFGQGTKVEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC.
>11W5 heavy chain: SEQ ID NO: 31
>11W5 light chain (SEQ ID NO: 65): 
DIVLTQSPASLAVSPGQRATITCRASESVDSYGNSFMHWYQQKPGQPPKLLIYHASNLES
GVPARFSGSGSRTDFTLTIDPVEADDAANYYCQQNNEDPWTFGQGTKVEIKRTVAAPSV
 
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC.
>11W6 heavy chain: SEQ ID NO: 31
>11W6 light chain (SEQ ID NO: 66): 
DIVLTQSPASLAVSPGQRATITCRASESVDSYGASFMHWYQQKPGQPPKLLIYYASNLES
GVPARFSGSGSRTDFTLTIDPVEADDAANYYCQQNNEDPWTFGQGTKVEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC.
>11W7 heavy chain (SEQ ID NO: 67): 
QVQLVQSGAEVKKPGASVKLSCKASGYTFTSYGIHWVRQAPGQGLEWMGELNPSNGRT
NYNEKFKSRVTLTRDKSTSTAYMQVSSLRSEDTAVYYCARWAYDALDYWGQGTLVTV
SSASTKGPSVFPLAPCSRSTSESTAALGCLVKDYFPEPVTVSWNSGALTSGVHTFPAVLQS
SGLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQFNS
TYRVVSVLTVLIIQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFLYSRLTVDKSR
WQEGNVFSCSVMHEALHNHYTQKSLSLSLGK.
>11W7 light chain (SEQ ID NO: 68): 
DIVLTQSPASLAVSPGQRATITCRASESVDSYGASFMHWYQQKPGQPPKLLIYHASNLES
GVPARFSGSGSRTDFTLTIDPVEADDAANYYCQQNNEDPWTFGQGTKVEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC.
>11W8 heavy chain (SEQ ID NO: 69): 
QVQLVQSGAEVKKPGASVKLSCKASGYTFTSYGIHWVRQAPGQGLEWMGELNPSNGRT
NYNEKFKSRVTLTRDKSTSTAYMQVSSLRSEDTAVYYCARWAYDAMDYWGQGTLVTV
SSASTKGPSVFPLAPCSRSTSESTAALGCLVKDYFPEPVTVSWNSGALTSGVHTFPAVLQS
SGLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQFNS
TYRVVSVLTVLHQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFLYSRLTVDKSR
 WQEGNVFSCSVMHEALHNHYTQKSLSLSLGK.
>11W8 light chain: SEQ ID NO: 65
>11W9 heavy chain (SEQ ID NO: 70): 
EVQLVQSGAEVKKPGASVKLSCKASGYTFTSYWIHWVRQAPGQGLEWMGELNPSNGR
TNYNEKFKSRVTLTVDKSTSTAYMEVSSLTSEDTAVYYCARWAYDAMDYWGQGTLVT
VSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLIIQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVMHEALHNHYTQKSLSLSLGK.
>11W9 light chain: SEQ ID NO: 64
>11W10 heavy chain: SEQ ID NO: 70
>11W10 light chain (SEQ ID NO: 71): 
DIVLTQSPASLAVSPGQRATITCRASESVDSYGNSFMHWYQQKPGQPPKLLIYYASNLES
GVPARFSGSGSRTDFTLTINPVEADDAANYYCQQNNEDPWTFGQGTKVEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC.
>12W1 heavy chain (SEQ ID NO: 32): 
QVQLVQSGAEVKKPGASVKVSCKASGYAFSNSWMNWVRQAPGQGLEWMGRIYPGDG
DINYNGKFKGRVTMTRDTSISTAYMELSSLRSDDTAVYYCARGGRDEGSMDYWGQGTL
VTVSSASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCPAPEFL
GGPSVFLFPPKPKDTLMISRTPEVTCVVVDVSQEDPEVQFNWYVDGVEVHNAKTKPREE
QFNSTYRVVSVLTVLHQDWLNGKEYKCKVSNKGLPSSIEKTISKAKGQPREPQVYTLPPS
QEEMTKNQVSLTCLVKGFYPSDIAVEWESNGQPENNYKTTPPVLDSDGSFFLYSRLTVD
KSRWQEGNVFSCSVMHEALHNHYTQKSLSLSLGK.
>12W1 light chain (SEQ ID NO: 33): 
DIQMTQSPSSLSASVGDRVTITCKASQNVGINVAWYQQKPGKAPKLLIYSASYRYSGVPD
RFSGSGSGTDFTLTISNLQSEDFATYFCQQYYSHFLFTFGQGTKVEIKRTVAAPSVFIFPPS
 
DEQLKSGTASVVCLLNNFYPREAKVQWKVDNALQSGNSQESVTEQDSKDSTYSLSSTLT
LSKADYEKHKVYACEVTHQGLSSPVTKSFNRGEC.
>12W2 heavy chain (SEQ ID NO: 34): 
QVQLVQSGAEVKKPGASVKVSCKASGYAFSNSWMNWVRQAPGQGLEWMGRIYPGDG
DINYNGKFKGRVTMTRDTSSSIAYMELSSLRSDDTAVYYCARGGRDEGSMDYWGQGTL
VTVSSASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCPAPEFL
GGPSVFLFPPKPKDTLMISRTPEVTCVVVDVSQEDPEVQFNWYVDGVEVHNAKTKPREE
QFNSTYRVVSVLTVLIIQDWLNGKEYKCKVSNKGLPSSIEKTISKAKGQPREPQVYTLPPS
QEEMTKNQVSLTCLVKGFYPSDIAVEWESNGQPENNYKTTPPVLDSDGSFFLYSRLTVD
KSRWQEGNVFSCSVMHEALHNHYTQKSLSLSLGK.
>12W2 light chain (SEQ ID NO: 35): 
DIVMTQSPSSLSASVGDRVTVTCKASQNVGINVAWYQQKPGQAPKPLIYSASYRYSGVP
DRFSGSGSGTDFTLTISSLQSEDFAEYYCQQYYSHFLFTFGQGTKVEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC.
>12W3 heavy chain (SEQ ID NO: 36): 
QVQLVQSGAEVKKPGASVKVSCKASGYAFSNSWMNWVRQAPGKGLEWMGRIYPGDG
DINYNGKFKGRVTMTADTSSSTAYMELSSLRSDDTAVYYCARGGRDEGSMDYWGQGT
LVTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK.
>12W3 light chain: SEQ ID NO: 35
>12W4 heavy chain (SEQ ID NO: 72): 
QVQLVQSGAEVKKPGASVKVSCKASGYAFSNSWMNWVRQAPGKGLEWMGRIYPGDG
DINYNGKFKGRVTMTADTSSSTAYMELSSLRSDDTAVYYCARGGRDEGSHDYWGQGT
LVTVSSASTKGPSVFPLAPCSRSTSESTAALGCLVKDYFPEPVTVSWNSGALTSGVHTFP
 
AVLQSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK.
>12W4 light chain (SEQ ID NO: 73): 
DIVMTQSPSSLSASVGDRVTVTCKASQNVGINVAWYQQKPGQAPKPLIYSASYRYSGVP
DRFSGSGSGTDFTLTISSLQSEDFAEYYCKQYYSHFLFSFGQGTKVEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC.
>12W5 heavy chain (SEQ ID NO: 74): 
QVQLVQSGAEVKKPGASVKVSCKASGYAFSNSWMDWVRQAPGKGLEWMGRIYPEDG
DINYNGKFKGRVTMTADTSSSTAYMELSSLRSDDTAVYYCARGGRDEGSMDYWGQGT
LVTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK.
>12W5 light chain (SEQ ID NO: 75): 
DIVMTQSPSSLSASVGDRVTVTCKASQNVGINVAWYQQKPGQAPKPLIYSASYRESGVP
DRFSGSGSGTDFTLTISSLQSEDFAEYYCQQYYSHFLTFGQGTKVEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC.
>12W6 heavy chain: SEQ ID NO: 36
>12W6 light chain (SEQ ID NO: 76): 
DIVMTQSPSSLSASVGDRVTVTCKASQNRGINVAWYQQKPGQAPKPLIYSASYRESGVP
DRFSGSGSGTDFTLTISSLQSEDFAEYYCQQYYSHFLTFGQGTKVEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC.
>12W7 heavy chain (SEQ ID NO: 77): 
QVQLVQSGAEVKKPGASVKVSCKASGYAFSNSWMDWVRQAPGKGLEWMGRIYPGDG
DINYNGKFKGRVTMTADTSSSTAYMELSSLRSDDTAVYYCARGGRDEGSMDYWGQGT
LVTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK.
>12W7 light chain (SEQ ID NO: 78): 
DIVMTQSPSSLSASVGDRVTVTCKASQNRGINVAWYQQKPGQAPKPLIYSASYRESGVP
DRFSGSGSGTDFTLTISSLQSEDFAEYYCQQYYSHRLFTFGQGTKVEIKRTVAAPSVFIFPP
SDEQLKSGTASVVCLLNNFYPREAKVQWKVDNALQSGNSQESVTEQDSKDSTYSLSSTL
TLSKADYEKHKVYACEVTHQGLSSPVTKSFNRGEC.
>12W8 heavy chain: SEQ ID NO: 36
>12W8 light chain: SEQ ID NO: 78
>12W9 heavy chain (SEQ ID NO: 79): 
QVQLVQSGAEVKKPGASVKVSCKASGYAFSNSWMNWVRQAPGKGLEWMGRIYPEDG
DINYNGKFKGRVTMTADTSSSTAYMELSSLRSDDTAVYYCARGGRDEGSMDYWGQGT
LVTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK.
>12W9 light chain: SEQ ID NO: 78
>12W10 heavy chain: SEQ ID NO: 74
>12W10 light chain: SEQ ID NO: 78

### Example 2. Preparation of Fully Human Antibodies Targeting MASP2

Peripheral blood mononuclear cells (PBMCs) were isolated from normal human blood using Ficoll-Paque density gradient separation solution (purchased from GE Company, Cat. No.: 17144003S). Total RNA was extracted from the isolated PBMCs using conventional methods. The extracted total RNA was reverse-transcribed into cDNA using a reverse transcription kit (purchased from TaKaRa Company, Cat. No.: 6210A). Based on the sequence similarity of the heavy chain and light chain germline genes, degenerate primers were designed at the front of the V region and the back of the first constant region of the heavy chain and light chain, respectively. After PCR, the heavy chain variable region gene fragment and light chain variable region gene fragment of the antibody were obtained. The corresponding human antibody gene phage display library was constructed, and library screening and monoclonal selection were performed according to the method described in Example 1.1. The selected positive clones were further subjected to antibody affinity maturation to obtain antibodies 20W30, 20W51, and 20W78. The amino acid sequences of the antibodies are shown in Table 5.

**Table 5. Heavy chain/light chain sequences of fully human antibodies targeting MASP2**

| | | |
|---|---|---|
| 20W30 | Heavy chain | EVQLVESGGGVVQPGRSLRLSCAASGFTFPSYAMHWVRQAPGKGLEWVAVIS YDLSNKYYADSVKGRFTISRDNSKNTLYLQMNSLRAEDTAVYYCAKSRRVYY YYGMDVWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKPS NTKVDKRVESKYGPPCPPCPAPEFLGGPSVFLFPPKPKDTLMISRTPEVTCVVVD VSQEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFS CSVMHEALHNHYTQKSLSLSLGK(SEQ ID NO: 37) |
| | Light chain | SYVLTQPPSVSVSPGQTASITCSAGKLGDKFASWYQQKPGQSPVLVIYQDSKRG SGIPERFSGSNSGNTATLTISGTQAMDEADYYCQAWDSSGYVFGTGTKVTVLG QPKAAPSVTLFPPSSEELQANKATLVCLISDFYPGAVTVAWKADSSPVKAGVET TTPSKQSNNKYAASSYLSLTPEQWKSHRSYSCQVTHEGSTVEKTVAPTECS (SEQ ID NO: 38) |
| 20W51 | Heavy chain | EVQLVESGGGVVQPGRSLRLSCAASGFTFPSYAMYWVRQAPGKGLEWVAVIS YDLSNKLYADSVKGRFTISRDNSKNTLYLQMNSLRAEDTAVYYCAKSRRQYY YYGMDVWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKPS NTKVDKRVESKYGPPCPPCPAPEFLGGPSVFLFPPKPKDTLMISRTPEVTCVVVD VSQEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFS CSVMHEALHNHYTQKSLSLSLGK(SEQ ID NO: 39) |
| | Light chain | SYVLTQPPSVSVSPGQTASITCSGGKLGDKFASWYQQKPGQSPVLVIYQDSKRG RGIPERFSGSNSGNTATLTISGTQAMDEADYYCQAWDSSTYVFGTGTKVTVLG QPKAAPSVTLFPPSSEELQANKATLVCLISDFYPGAVTVAWKADSSPVKAGVET TTPSKQSNNKYAASSYLSLTPEQWKSHRSYSCQVTHEGSTVEKTVAPTECS(SE Q ID NO: 40) |
| 20W78 | Heavy chain | EVQLVESGGGVVQPGRSLRLSCAASGFTFPSYAMHWVRQAPGKGLEWVAVIS YDLSNKYYADSVKGRFTISRDNSKNTLYLQMNSLRAEDTAVYYCAKSRRVYY YYGMDVWGQGTTVTVSSASTKGPSVFPLAPCSRSTSESTAALGCLVKDYFPEP VTVSWNSGALTSGVHTFPAVLQSSGLYSLSSVVTVPSSSLGTKTYTCNVDHKPS NTKVDKRVESKYGPPCPPCPAPEFLGGPSVFLFPPKPKDTLMISRTPEVTCVVVD VSQEDPEVQFNWYVDGVEVHNAKTKPREEQFNSTYRVVSVLTVLHQDWLNG KEYKCKVSNKGLPSSIEKTISKAKGQPREPQVYTLPPSQEEMTKNQVSLTCLVK GFYPSDIAVEWESNGQPENNYKTTPPVLDSDGSFFLYSRLTVDKSRWQEGNVFS CSVMHEALHNHYTQKSLSLSLGK(SEQ ID NO: 37) |
| | Light chain | SYVLTQPPSVSVSPGQTASITCSGYKLGDKFASWYQQKPGQSPVLVIYQDSKRG SGIPERFSGSNSGNTATLTISGTQAMDEADYYCQAWDSSGYVFGTGTKVTVLG QPKAAPSVTLFPPSSEELQANKATLVCLISDFYPGAVTVAWKADSSPVKAGVET TTPSKQSNNKYAASSYLSLTPEQWKSHRSYSCQVTHEGSTVEKTVAPTECS(SE Q ID NO: 41) |

### Example 3. Evaluation of the Binding Ability of Anti-MASP2 Antibodies

### 3.1 Determination of antibody affinity by kinetic level (ForteBio)

The affinity constant (KD) of the antibody binding to human MASP2 was determined by kinetic level. The antibody was immobilized on a Protein A sensor, and serially diluted human MASP2 protein was subjected to equilibration (baseline), association, and dissociation steps, followed by regeneration and neutralization of the Protein A sensor over multiple cycles. The affinity constant (KD), association rate constant (Kon), and dissociation rate constant (Kdis) of the antibodies for MASP2 were then analyzed. The experimental results showed that the affinity KD of the 11W and 12W series antibodies of the present disclosure for MASP2 protein was less than 10⁻⁸ M. It can be seen that the affinity of the 11W and 12W series antibodies of the present disclosure for binding to human MASP2 protein was superior to that of the control antibody Narsoplimab (Table 6).

**Table 6. Affinity test results of 11W and 12W series antibodies binding to human MASP2 protein**

| Antibody ID | KD (M) | Kon (1/Ms) | Kdis (1/s) |
|---|---|---|---|
| 11W0 | 1.57×10⁻⁹ | 1.34×10⁵ | 2.10×10⁻⁴ |
| 11W1 | 3.03×10⁻⁹ | 1.57×10⁵ | 4.77×10⁻⁴ |
| 11W2 | 2.82×10⁻⁹ | 1.58×10⁵ | 4.45×10⁻⁴ |
| 11W3 | 2.56×10⁻⁹ | 1.14×10⁵ | 2.92×10⁻⁴ |
| 12W0 | 7.63×10⁻⁹ | 1.24×10⁵ | 9.47×10⁻⁴ |
| 12W1 | 9.03×10⁻⁹ | 1.43×10⁵ | 1.29×10⁻³ |
| 12W2 | 9.70×10⁻⁹ | 1.58×10⁵ | 1.53×10⁻³ |
| 12W3 | 7.51×10⁻⁹ | 1.34×10⁵ | 1.01×10⁻³ |
| Narsoplimab | 4.04×10⁻⁸ | 1.56×10⁵ | 6.31×10⁻³ |

### 3.2 Determination of antibody affinity by ELISA

The relative binding activity of each antibody to human MASP2 protein was determined by ELISA. The specific procedure was as follows: recombinant human MASP2 protein (2 µg/mL) was coated onto an ELISA plate at 100 µL/well and incubated at 4°C overnight. Then, the wells were blocked with PBST (containing 0.05% Tween-20) containing 1% BSA at 37°C for 2 hours (200 µL/well), and washed three times with PBST. Each antibody was serially diluted with PBST containing 1% BSA and added to the ELISA plate (100 µL/well). The working concentrations of the antibody were prepared by 4-fold serial dilution from a starting concentration of 10,000 ng/mL, yielding 8 concentration points. After incubation at 37°C for 1 hour, the wells were washed three times with PBST. Then, anti-human IgG-FC-HRP (JACSON; 109-035-170) was added at 100 µL/well, incubated at 37°C for 1 hour, and washed three times with PBST. Subsequently, 100 µL of TMB (SURMOPICS) was added for reaction, terminated with 2 M HCl, and the OD value was measured at 450 nm-570 nm using a microplate reader.

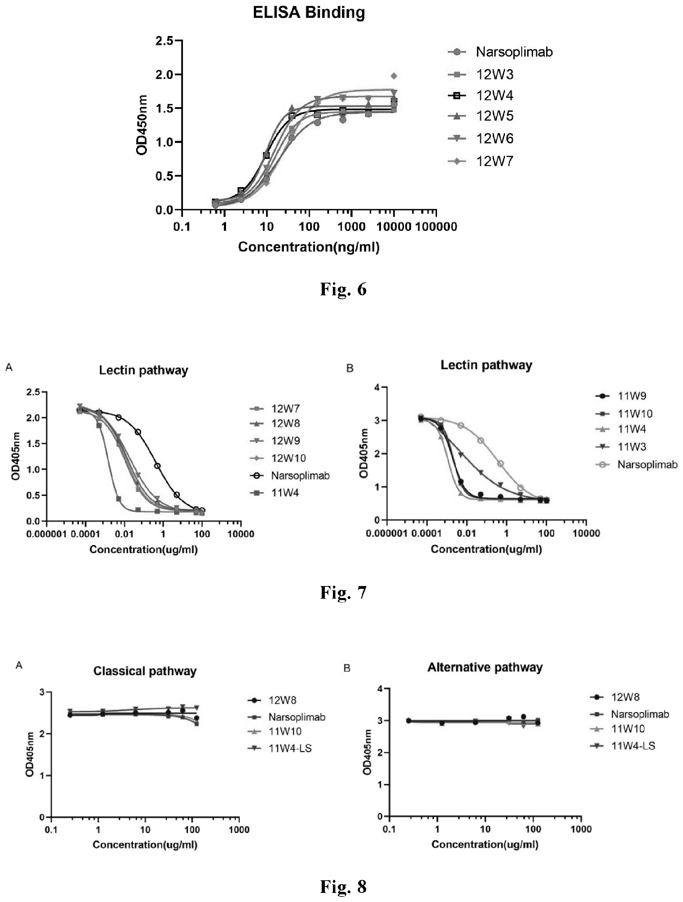

The experimental results are shown in Table 7 and Figure 6. It can be seen that the affinity of the 12W and 20W series antibodies of the present disclosure for binding to human MASP2 protein was superior to that of the control antibody Narsoplimab.

**Table 7. Affinity test results of 20W series antibodies for binding to MASP2 protein**

| Antibody ID | EC₅₀ (µg/mL) |
|---|---|
| 20W30 | 0.045 |
| 20W78 | 0.053 |
| Narsoplimab | 0.108 |

### Example 4. Specificity of Anti-MASP2 Antibodies in Inhibiting the Complement Lectin Pathway in Vitro

### 4.1 Determination of complement lectin pathway MAC

The inhibitory effect of candidate antibodies was determined using the WIESLAB^{®} complement lectin pathway screening kit (Complement System MBL pathway: COMPLMP320RUO). The plate was pre-coated with mannan as an activator of the MBL pathway. Antibodies were serially diluted with assay buffer, and human complement serum (Quidel, A112) was also diluted with assay buffer, then incubated on ice for 30 minutes. The antibody-serum mixture was added to the plate and incubated at 37°C for 120 minutes. After washing the plate, deposited MAC (membrane attack complex, C5b-9) was detected using an alkaline phosphatase-labeled specific antibody.

The experimental results are shown in Tables 8 and 9 and Figure 7. It can be seen that the inhibitory activity of the antibodies of the present disclosure against the complement lectin pathway was superior to that of the control antibody Narsoplimab. In addition, 11W1 and 12WO exhibited inhibitory activity comparable to that of the control antibody Narsoplimab.

**Table 8. Inhibition of complement lectin pathway by anti-MASP2 antibodies**

| Antibody ID | IC₅₀ (µg/mL) |
|---|---|
| 20W30 | 0.221 |
| 20W51 | 0.193 |
| 20W78 | 0.188 |
| Narsoplimab | 0.911 |

**Table 9.1 Inhibition of complement lectin pathway by anti-MASP2 antibodies**

| Antibody ID | IC₅₀ (µg/mL) |
|---|---|
| 11WO | 0.470 |
| 11W3 | 0.063 |

**Table 9.2 Inhibition of complement lectin pathway by anti-MASP2 antibodies**

| Antibody ID | IC₅₀ (µg/mL) |
|---|---|
| 12W4 | 0.630 |
| 12W5 | 0.289 |
| 12W6, 12W7, 12W8, 12W9, 12W10 | <0.1 |
| 11W9, 11W10 | <0.01 |

### 4.2 Determination of complement classical pathway and alternative pathway MAC

The selectivity of candidate antibodies for the complement system was determined using the WIESLAB^{®} complement classical pathway screening kit (Complement System Classical pathway: COMPLCP310RUO) and the alternative pathway screening kit (Complement System Alternative pathway: COMPLAP330RUO). The plate was pre-coated with IgM as an activator of the classical pathway and LPS as an activator of the alternative pathway. Antibodies were serially diluted with assay buffer, and human complement serum (Quidel, Al12) was also diluted with assay buffer, then incubated on ice for 30 minutes. The antibody-serum mixture was added to the plate and incubated at 37°C for 120 minutes. After washing the plate, deposited MAC was detected using an alkaline phosphatase-labeled specific antibody.

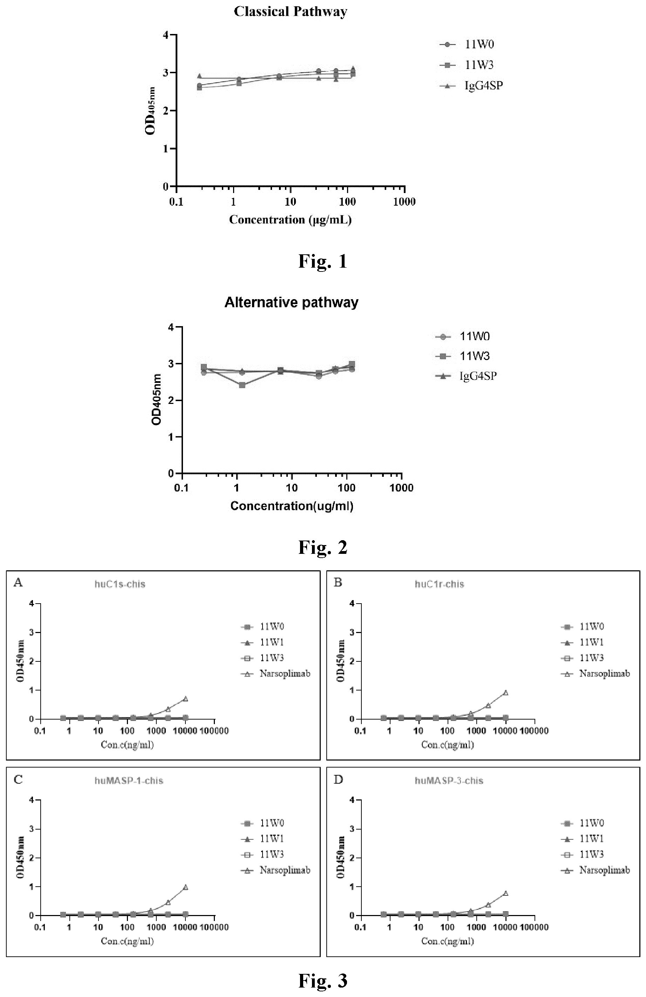

The experimental results are shown in Figures 1 and 2. It can be seen that the anti-MASP2 antibodies of the present disclosure did not substantially inhibit the complement classical and alternative pathways, but specifically inhibited the complement lectin pathway.

### Example 5. Determination of Cross-Reactivity of Anti-MASP2 Antibodies

### 5.1 MASP2 species cross-reactivity test

The relative binding activity of each antibody to MASP2 protein was determined by ELISA. Recombinant human, mouse, and monkey MASP2 proteins (2 µg/mL) were coated on an ELISA plate at 100 µL/well and incubated at 4°C overnight. Then, the wells were blocked with PBST (containing 0.05% Tween-20) containing 1% BSA at 37°C for 2 hours (200 µL/well), and washed three times with PBST. Candidate antibodies were serially diluted with PBST containing 1% BSA and added to the ELISA plate (100 µL/well). The working concentrations of the antibody were prepared by 4-fold serial dilution from a starting concentration of 10,000 ng/mL, yielding 8 concentration points. Narsoplimab was used as a positive control, and an isotype IgG antibody was used as a negative control. After incubation at 37°C for 1 hour, the wells were washed three times with PBST. Then, anti-human IgG-FC-HRP (JACSON; 109-035-170) was added at 100 µL/well, incubated at 37°C for 1 hour, and washed three times with PBST. Subsequently, 100 µL of TMB (SURMOPICS) was added for reaction, terminated with 2 M HCl, and the OD value was measured at 450 nm-570 nm using a microplate reader. The experimental results are shown in Table 10.

**Table 10. Binding of anti-MASP2 antibodies to MASP2 proteins of various species**

| Antibody ID | Human MASP2 protein | Monkey MASP2 protein | Mouse MASP2 protein |
|---|---|---|---|
| 20W30 | √ | √ | √ |
| 20W78 | √ | √ | √ |
| 11WO | √ | √ | - |
| 11W1 | √ | √ | - |
| 11W3 | √ | √ | - |
| 11W10 | √ | √ | - |
| 12W6 | √ | - | √ |
| 12W8 | √ | - | √ |
| Narsoplimab | √ | √ | √ |
| IgG4SP | × | × | × |

| | | | |
|---|---|---|---|
| Note: "√" indicates binding activity to the antigen protein; "×" indicates no binding activity to the antigen protein; Mouse MASP2 protein refers to MASP2 protein from mouse. | | | |

### 5.2 Complement family member cross-reactivity test

The relative binding activity of each antibody to complement family members C1r, C1s, MASP1, and MASP3 was determined by ELISA. C1r, C1s, MASP1, and MASP3 proteins (2 µg/mL) were coated onto an ELISA plate at 100 µL/well and incubated at 4°C overnight. Then, the wells were blocked with PBST (containing 0.05% Tween-20) containing 1% BSA at 37°C for 2 hours (200 µL/well), and washed three times with PBST. Candidate antibodies were serially diluted with PBST containing 1% BSA and added to the ELISA plate (100 µL/well). The working concentrations of the antibody were prepared by 4-fold serial dilution from a starting concentration of 10,000 ng/mL, yielding 8 concentration points. Narsoplimab was used as a positive control. After incubation at 37°C for 1 hour, the wells were washed three times with PBST. Then, anti-human IgG-FC-HRP (JACSON; 109-035-170) was added at 100 µL/well, incubated at 37°C for 1 hour, and washed three times with PBST. Subsequently, 100 µL of TMB (SURMOPICS) was added for reaction, terminated with 2 M HCl, and the OD value was measured at 450 nm-570 nm using a microplate reader. The experimental results are shown in Figure 3. It can be seen that the antibodies of the present disclosure showed substantially no binding activity to complement family members.

### Example 6. Long-acting Modification of Anti-MASP2 Antibodies

To improve the pharmacokinetic (PK) characteristics of the humanized antibodies, mutations were introduced into the Fc region to extend antibody half-life. The M428L/N434S mutation (LS mutation) and the M252Y/S254T/T256E mutation (YTE mutation) have been shown to prolong antibody half-life by increasing FcRn binding affinity (Booth et al., mAbs 10(7): 1098-1110, 2018; Dall'acqua WF et al., J Biol Chem 281:23514-24, 2006). Therefore, humanized antibodies with extended half-life were generated. The heavy chain/light chain amino acid sequences of each antibody are as follows:
>20W30-LS heavy chain (SEQ ID NO: 42): 
EVQLVESGGGVVQPGRSLRLSCAASGFTFPSYAMHWVRQAPGKGLEWVAVISYDLSNK
YYADSVKGRFTISRDNSKNTLYLQMNSLRAEDTAVYYCAKSRRVYYYYGMDVWGQGT
TVTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLTV
DKSRWQEGNVFSCSVLHEALHSHYTQKSLSLSLGK.
>20W30-LS light chain: SEQ ID NO: 38
>20W30-YTE heavy chain (SEQ ID NO: 43): 
EVQLVESGGGVVQPGRSLRLSCAASGFTFPSYAMHWVRQAPGKGLEWVAVISYDLSNK
YYADSVKGRFTISRDNSKNTLYLQMNSLRAEDTAVYYCAKSRRVYYYYGMDVWGQGT
TVTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPEF
LGGPSVFLFPPKPKDTLYITREPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLTV
DKSRWQEGNVFSCSVMHEALHNHYTQKSLSLSLGK.
>20W30-YTE light chain: SEQ ID NO: 38
>11W3-LS heavy chain (SEQ ID NO: 44): 
QVQLVQSGAEVKKPGASVKLSCKASGYTFTSYWIHWVRQAPGQGLEWMGELNPSNGR
TNYNEKFKSRVTLTRDKSTSTAYMQVSSLRSEDTAVYYCARWAYDAMDYWGQGTLVT
VSSASTKGPSVFPLAPCSRSTSESTAALGCLVKDYFPEPVTVSWNSGALTSGVHTFPAVL
 
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVLHEALHSHYTQKSLSLSLGK.
>11W3-LS light chain: SEQ ID NO: 30
>11W4-LS heavy chain (SEQ ID NO: 44): 
QVQLVQSGAEVKKPGASVKLSCKASGYTFTSYWIHWVRQAPGQGLEWMGELNPSNGR
TNYNEKFKSRVTLTRDKSTSTAYMQVSSLRSEDTAVYYCARWAYDAMDYWGQGTLVT
VSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLIIQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVLHEALHSHYTQKSLSLSLGK.
>11W4-LS light chain (SEQ ID NO: 64): 
DIVLTQSPASLAVSPGQRATITCRASESVDSYGNSFMHWYQQKPGQPPKLLIYYASNLES
GVPARFSGSGSRTDFTLTIDPVEADDAANYYCQQNNEDPWTFGQGTKVEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC.
>12W3-LS heavy chain (SEQ ID NO: 45): 
QVQLVQSGAEVKKPGASVKVSCKASGYAFSNSWMNWVRQAPGKGLEWMGRIYPGDG
DINYNGKFKGRVTMTADTSSSTAYMELSSLRSDDTAVYYCARGGRDEGSMDYWGQGT
LVTVSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYTLPP
SQEEMTKNQVSLTCLVKGFYPSDIAVEWESNGQPENNYKTTPPVLDSDGSFFLYSRLTV
DKSRWQEGNVFSCSVLHEALHSHYTQKSLSLSLGK.
>12W3 light chain: SEQ ID NO: 35
>11W5-LS heavy chain (SEQ ID NO: 44): 
QVQLVQSGAEVKKPGASVKLSCKASGYTFTSYWIHWVRQAPGQGLEWMGELNPSNGR
TNYNEKFKSRVTLTRDKSTSTAYMQVSSLRSEDTAVYYCARWAYDAMDYWGQGTLVT
VSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLIIQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVLHEALHSHYTQKSLSLSLGK.
>11W5-LS light chain: SEQ ID NO: 65
>11W6-LS heavy chain (SEQ ID NO: 44): 
QVQLVQSGAEVKKPGASVKLSCKASGYTFTSYWIHWVRQAPGQGLEWMGELNPSNGR
TNYNEKFKSRVTLTRDKSTSTAYMQVSSLRSEDTAVYYCARWAYDAMDYWGQGTLVT
VSSASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEFLGG
PSVFLFPPKPKDTLMISRTPEVTCVVVDVSQEDPEVQFNWYVDGVEVHNAKTKPREEQF
NSTYRVVSVLTVLIIQDWLNGKEYKCKVSNKGLPSSIEKTISKAKGQPREPQVYTLPPSQE
EMTKNQVSLTCLVKGFYPSDIAVEWESNGQPENNYKTTPPVLDSDGSFFLYSRLTVDKS
RWQEGNVFSCSVLHEALHSHYTQKSLSLSLGK.
>11W6-LS light chain: SEQ ID NO: 66
>11W7-LS heavy chain (SEQ ID NO: 80): 
QVQLVQSGAEVKKPGASVKLSCKASGYTFTSYGIHWVRQAPGQGLEWMGELNPSNGRT
NYNEKFKSRVTLTRDKSTSTAYMQVSSLRSEDTAVYYCARWAYDALDYWGQGTLVTV
SSASTKGPSVFPLAPCSRSTSESTAALGCLVKDYFPEPVTVSWNSGALTSGVHTFPAVLQS
SGLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQFNS
TYRVVSVLTVLIIQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFLYSRLTVDKSR
WQEGNVFSCSVLHEALHSHYTQKSLSLSLGK.
>11W7-LS light chain: SEQ ID NO: 68
>11W8-LS heavy chain (SEQ ID NO: 81): 
QVQLVQSGAEVKKPGASVKLSCKASGYTFTSYGIHWVRQAPGQGLEWMGELNPSNGRT
NYNEKFKSRVTLTRDKSTSTAYMQVSSLRSEDTAVYYCARWAYDAMDYWGQGTLVTV
SSASTKGPSVFPLAPCSRSTSESTAALGCLVKDYFPEPVTVSWNSGALTSGVHTFPAVLQS
SGLYSLSSVVTVPSSSLGTKTYTCNVDHKPSNTKVDKRVESKYGPPCPPCPAPEFLGGPS
VFLFPPKPKDTLMISRTPEVTCVVVDVSQEDPEVQFNWYVDGVEVHNAKTKPREEQFNS
TYRVVSVLTVLIIQDWLNGKEYKCKVSNKGLPSSIEKTISKAKGQPREPQVYTLPPSQEE
MTKNQVSLTCLVKGFYPSDIAVEWESNGQPENNYKTTPPVLDSDGSFFLYSRLTVDKSR
WQEGNVFSCSVLHEALHSHYTQKSLSLSLGK.
>11W8-LS light chain: SEQ ID NO: 65

The affinity constant (KD) of the antibody binding to FcRn was determined by kinetic level (ForteBio). Human FcRn was immobilized on a Protein A sensor, and serially diluted antibodies were subjected to equilibration (baseline), association, and dissociation steps, followed by regeneration and neutralization of the Protein A sensor over multiple cycles. The affinity constant (KD), association rate constant (Kon), and dissociation rate constant (Kdis) of the antibodies for human FcRn were then analyzed. The results of the affinity test of each antibody for human FcRn are shown in Tables 11 and 12. It can be seen that 20W30 showed increased affinity for FcRn following LS mutation or YTE mutation modifications, with the LS mutation of a better effect (Table 11); 11W3 showed an approximately 10-fold increase in affinity for FcRn following LS mutation modification (Table 12).

**Table 11. Affinity test results of anti-MASP2 antibodies for human FcRn**

| Antibody ID | KD(M) | Kon(1/Ms) | Kdis(1/s) |
|---|---|---|---|
| 20W30 | 5.49×10⁻⁹ | 1.99×10⁶ | 1.09×10⁻² |
| 20W30-LS | 1.67×10⁻⁹ | 1.79×10⁶ | 2.99×10⁻³ |
| 20W30-YTE | 2.21×10⁻⁹ | 1.41×10⁶ | 3.11×10⁻³ |

**Table 12. Affinity test results of anti-MASP2 antibodies for human FcRn**

| Antibody ID | KD(M) | Kon(1/Ms) | Kdis(1/s) |
|---|---|---|---|
| 11W3-LS | 4.09×10⁻⁹ | 2.20×10⁶ | 8.98×10⁻³ |
| 11W3 | 4.42×10⁻⁸ | 1.27×10⁶ | 5.59×10⁻² |

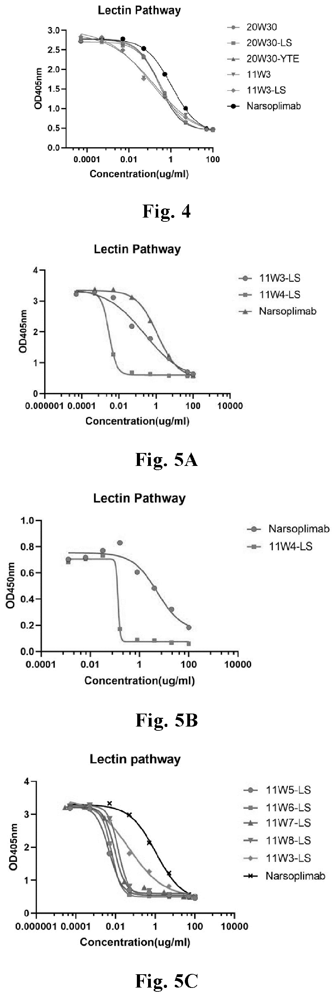

Following the method described in Example 4, the effect of long-acting modified antibodies on complement lectin pathway MAC was determined. The results are shown in Figures 4 and 5. It can be seen that the long-acting modification did not substantially affect the inhibitory activity of the antibodies. The inhibitory activity of the modified antibodies remained at a level comparable to that before modification, and was superior to that of the control antibody Narsoplimab. Figures 5A to 5C show the inhibitory activity of other modified antibodies. Figures 5A and 5B show that antibody 11W4-LS exhibited inhibitory activity against the complement lectin pathway far exceeding that of Narsoplimab, with the inhibitory activity increased by nearly 100-fold in the presence of 1% human serum (Figure 5A), and it did not substantially affect the alternative and classical pathways (Figure 8).

Following the method described in Example 5, the species cross-reactivity of long-acting modified antibodies against MASP2 was determined, and the results are shown in Table 13. It can be seen that neither LS mutation nor YTE mutation affected the species cross-reactivity of the antibodies.

**Table 13. Binding of anti-MASP2 antibodies to MASP2 proteins of various species**

| Antibody ID | Human MASP2 protein | Monkey MASP2 protein | Mouse MASP2 protein |
|---|---|---|---|
| 20W30 | √ | √ | √ |
| 20W30-LS | √ | √ | √ |
| 20W30-YTE | √ | √ | √ |
| 11W3 | √ | √ | - |
| 11W3-LS | √ | √ | - |
| 11W4-LS | √ | √ | - |
| 12W3 | √ | √ | - |
| 12W3-LS | √ | √ | - |
| Narsoplimab | √ | √ | √ |
| IgG4SP | × | × | × |

| | | | |
|---|---|---|---|
| Note: Mouse MASP2 protein refers to MASP2 protein from mouse. | | | |

In addition, the binding of the 11W series antibodies to rat MASP2 protein was further tested. ELISA method was performed as follows: Biotinylated recombinant rat MASP2 protein (2 µg/mL) was coated on a streptavidin (SA) pre-coated ELISA plate at 100 µL/well and incubated at 4°C overnight. Then, candidate antibodies were serially diluted with PBS containing 2% BSA and added to the ELISA plate (100 µL/well). The working concentrations of the antibody were prepared by 4-fold serial dilution from a starting concentration of 10,000 ng/mL, yielding 8 concentration points. After incubation at 37°C for 1 hour, the wells were washed 5 times with PBST. Then, anti-human IgG (JACSON; 109-035-003) diluted 1:10000 in PBS containing 2% BSA was added at 100 µL/well, incubated at 37°C for 1 hour, and washed five times with PBST. Subsequently, 100 µL of TMB (SURMOPICS) was added for reaction, incubated at room temperature, terminated with 1 M H₂SO₄, and the OD value was measured at 450 nm using a microplate reader. The results showed that antibody 11W4-LS also showed binding activity to rat MASP2 protein.

### Example 7. Effect of Anti-MASP2 Antibodies on C4b Deposition

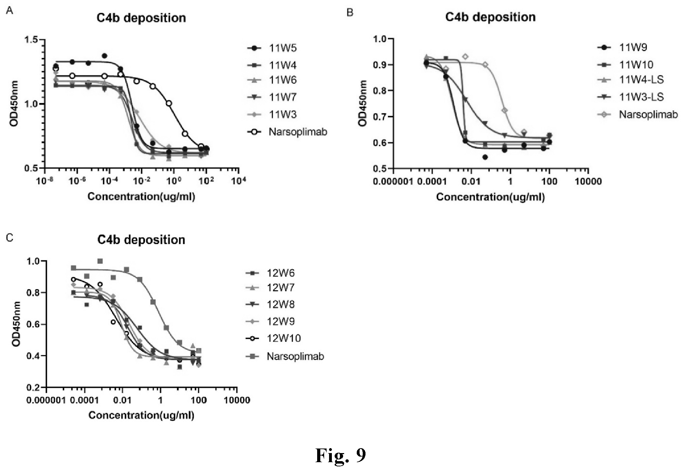

10 µg/mL of mannan (Sigma, M7504) was added to the wells of the microtiter plate, and coated overnight at 4°C. After washing the plate, 200 µL/well of blocking solution was added and blocked at room temperature for 2 hours. 1% of human complement serum (Creative Biolabs, CTS-006) was mixed with antibody dilutions, incubated on ice for 45 min, then added to the microtiter plate, and incubated at 37°C for about 2 hours. After washing the plate, 100 µL/well of 1:700 diluted detection antibody (ASSAYPRO, #11223-05021) was added and incubated at room temperature for 60 min. After washing the plate, 100 µL/well of 1:200 diluted peroxidase-streptavidin (R&D, DY998) was added and incubated at room temperature for 45 min. After washing the plate, TMB substrate solution was added and incubated at 37°C in the dark for about 2 min. Finally, 1 M of H₂SO₄ was added to stop the reaction, and the OD value was measured. The results are shown in Figure 9. It can be seen that the antibodies of the present disclosure were more effective in reducing C4b deposition in the blood. The calculated IC₅₀ values of the optimized 11W series antibodies shown in Figure 9 for inhibiting C4b deposition were all not higher than 0.01 µg/mL, demonstrating significantly improved inhibitory activity compared to that of Narsoplimab.

Although the present disclosure has been described with reference to one or more embodiments, it should be understood that the disclosure is not limited to these embodiments and that the description of the disclosure is intended to cover all alternatives, modifications and variations falling within the spirit and broad scope of the appended claims. All references cited in the present disclosure are incorporated by reference in their entirety into the present disclosure.

## Claims

1. An anti-MASP2 antibody or antigen-binding fragment thereof, the anti-MASP2 antibody or antigen-binding fragment thereof comprising a heavy chain variable region (VH) and a light chain variable region (VL), the VH comprising HCDR1, HCDR2 and HCDR3 regions, and the VL comprising LCDR1, LCDR2 and LCDR3 regions, wherein:
(i) the HCDR1, HCDR2 and HCDR3 regions have sequences identical to the HCDR1, HCDR2 and HCDR3 of a VH as shown in any one of SEQ ID NOs: 1-2, or sequences having up to 5, 4, 3, 2 or 1 mutation(s) compared thereto, and the LCDR1, LCDR2 and LCDR3 regions have sequences identical to the LCDR1, LCDR2 and LCDR3 of a VL as shown in any one of SEQ ID NOs: 3-5, or sequences having up to 5, 4, 3, 2 or 1 mutation(s) compared thereto;
(ii) the HCDR1, HCDR2 and HCDR3 regions have sequences identical to the HCDR1, HCDR2 and HCDR3 of a VH as shown in any one of SEQ ID NOs: 6-8 and 48-50, or sequences having up to 5, 4, 3, 2 or 1 mutation(s) compared thereto, and the LCDR1, LCDR2 and LCDR3 regions have sequences identical to the LCDR1, LCDR2 and LCDR3 of a VL as shown in any one of SEQ ID NOs: 9-11 and 51-55, or sequences having up to 5, 4, 3, 2 or 1 mutation(s) compared thereto; or
(iii) the HCDR1, HCDR2 and HCDR3 regions have sequences identical to the HCDR1, HCDR2 and HCDR3 of a VH as shown in any one of SEQ ID NOs: 12-15 and 56-59, or sequences having up to 5, 4, 3, 2 or 1 mutation(s) compared thereto, and the LCDR1, LCDR2 and LCDR3 regions have sequences identical to the LCDR1, LCDR2 and LCDR3 of a VL as shown in any one of SEQ ID NOs: 16-18 and 60-63, or sequences having up to 5, 4, 3, 2 or 1 mutation(s) compared thereto; the mutation is selected from insertions, deletions and/or substitutions that do not affect function, and the substitution is preferably a conservative amino acid substitution.

2. The antibody or antigen-binding fragment thereof according to claim 1, wherein the VH has HCDR1, HCDR2 and HCDR3 identical in sequence to those of the VH as shown in SEQ ID NO: 1, and the VL has LCDR1, LCDR2 and LCDR3 identical in sequence to those of the VL as shown in SEQ ID NO: 3 or 5;
the VH has HCDR1, HCDR2 and HCDR3 identical in sequence to those of the VH as shown in SEQ ID NO: 2, and the VL has LCDR1, LCDR2 and LCDR3 identical in sequence to those of the VL as shown in SEQ ID NO: 4;
the VH has HCDR1, HCDR2 and HCDR3 identical in sequence to those of the VH as shown in SEQ ID NO: 6, and the VL has LCDR1, LCDR2 and LCDR3 identical in sequence to those of the VL as shown in SEQ ID NO: 9;
the VH has HCDR1, HCDR2 and HCDR3 identical in sequence to those of the VH as shown in SEQ ID NO: 7, and the VL has LCDR1, LCDR2 and LCDR3 identical in sequence to those of the VL as shown in SEQ ID NO: 10 or 11;
the VH has HCDR1, HCDR2 and HCDR3 identical in sequence to those of the VH as shown in SEQ ID NO: 8, and the VL has LCDR1, LCDR2 and LCDR3 identical in sequence to those of the VL as shown in any one of SEQ ID NOs: 11 and 51-53;
the VH has HCDR1, HCDR2 and HCDR3 identical in sequence to those of the VH as shown in SEQ ID NO: 48, and the VL has LCDR1, LCDR2 and LCDR3 identical in sequence to those of the VL as shown in SEQ ID NO: 54;
the VH has HCDR1, HCDR2 and HCDR3 identical in sequence to those of the VH as shown in SEQ ID NO: 49, and the VL has LCDR1, LCDR2 and LCDR3 identical in sequence to those of the VL as shown in SEQ ID NO: 52;
the VH has HCDR1, HCDR2 and HCDR3 identical in sequence to those of the VH as shown in SEQ ID NO: 50, and the VL has LCDR1, LCDR2 and LCDR3 identical in sequence to those of the VL as shown in SEQ ID NO: 51 or 55;
the VH has HCDR1, HCDR2 and HCDR3 identical in sequence to those of the VH as shown in SEQ ID NO: 12, and the VL has LCDR1, LCDR2 and LCDR3 identical in sequence to those of the VL as shown in SEQ ID NO: 16;
the VH has HCDR1, HCDR2 and HCDR3 identical in sequence to those of the VH as shown in SEQ ID NO: 13, and the VL has LCDR1, LCDR2 and LCDR3 identical in sequence to those of the VL as shown in SEQ ID NO: 17;
the VH has HCDR1, HCDR2 and HCDR3 identical in sequence to those of the VH as shown in SEQ ID NO: 14, and the VL has LCDR1, LCDR2 and LCDR3 identical in sequence to those of the VL as shown in SEQ ID NO: 18;
the VH has HCDR1, HCDR2 and HCDR3 identical in sequence to those of the VH as shown in SEQ ID NO: 15, and the VL has LCDR1, LCDR2 and LCDR3 identical in sequence to those of the VL as shown in SEQ ID NO: 18 or 62;
the VH has HCDR1, HCDR2 and HCDR3 identical in sequence to those of the VH as shown in SEQ ID NO: 56, and the VL has LCDR1, LCDR2 and LCDR3 identical in sequence to those of the VL as shown in SEQ ID NO: 60;
the VH has HCDR1, HCDR2 and HCDR3 identical in sequence to those of the VH as shown in SEQ ID NO: 57, and the VL has LCDR1, LCDR2 and LCDR3 identical in sequence to those of the VL as shown in SEQ ID NO: 61; or,
the VH has HCDR1, HCDR2 and HCDR3 identical in sequence to those of the VH as shown in any one of SEQ ID NOs: 15 and 57-59, and the VL has LCDR1, LCDR2 and LCDR3 identical in sequence to those of the VL as shown in SEQ ID NO: 63.

3. The antibody or antigen-binding fragment thereof according to claim 1 or 2, wherein the VH has an amino acid sequence having at least 85%, 90%, 91%, 92%, 93%, 94%, 95%, 96%, 97%, 98%, 99% or 100% identity to a sequence as shown in any one of SEQ ID NOs: 1-2, and/or the VL has an amino acid sequence having at least 85%, 90%, 91%, 92%, 93%, 94%, 95%, 96%, 97%, 98%, 99% or 100% identity to a sequence as shown in any one of SEQ ID NOs: 3-5;
the VH has an amino acid sequence having at least 85%, 90%, 91%, 92%, 93%, 94%, 95%, 96%, 97%, 98%, 99% or 100% identity to a sequence as shown in any one of SEQ ID NOs: 6-8 and 48-50, and/or the VL has an amino acid sequence having at least 85%, 90%, 91%, 92%, 93%, 94%, 95%, 96%, 97%, 98%, 99% or 100% identity to a sequence as shown in any one of SEQ ID NOs: 9-11 and 51-55; or
the VH has an amino acid sequence having at least 85%, 90%, 91%, 92%, 93%, 94%, 95%, 96%, 97%, 98%, 99% or 100% identity to a sequence as shown in any one of SEQ ID NOs: 12-15 and 56-59, and/or the VL has an amino acid sequence having at least 85%, 90%, 91%, 92%, 93%, 94%, 95%, 96%, 97%, 98%, 99% or 100% identity to a sequence as shown in any one of SEQ ID NOs: 16-18 and 60-63.

4. The antibody or antigen-binding fragment thereof according to any one of claims 1-3, wherein the VH comprises the sequence as shown in SEQ ID NO: 1, and the VL comprises the sequence as shown in any one of SEQ ID NOs: 3-5;
the VH comprises the sequence as shown in SEQ ID NO: 2, and the VL comprises the sequence as shown in any one of SEQ ID NOs: 3-5;
the VH comprises the sequence as shown in SEQ ID NO: 6, and the VL comprises the sequence as shown in any one of SEQ ID NOs: 9-11 and 51-556;
the VH comprises the sequence as shown in SEQ ID NO: 7, and the VL comprises the sequence as shown in any one of SEQ ID NOs: 9-11 and 51-55;
the VH comprises the sequence as shown in SEQ ID NO: 8, and the VL comprises the sequence as shown in any one of SEQ ID NOs: 9-11 and 51-55;
the VH comprises the sequence as shown in SEQ ID NO: 48, and the VL comprises the sequence as shown in any one of SEQ ID NOs: 9-11 and 51-55;
the VH comprises the sequence as shown in SEQ ID NO: 49, and the VL comprises the sequence as shown in any one of SEQ ID NOs: 9-11 and 51-55;
the VH comprises the sequence as shown in SEQ ID NO: 50, and the VL comprises the sequence as shown in any one of SEQ ID NOs: 9-11 and 51-55;
the VH comprises the sequence as shown in SEQ ID NO: 12, and the VL comprises the sequence as shown in any one of SEQ ID NOs: 16-18 and 60-63;
the VH comprises the sequence as shown in SEQ ID NO: 13, and the VL comprises the sequence as shown in any one of SEQ ID NOs: 16-18 and 60-63;
the VH comprises the sequence as shown in SEQ ID NO: 14, and the VL comprises the sequence as shown in any one of SEQ ID NOs: 16-18 and 60-63;
the VH comprises the sequence as shown in SEQ ID NO: 15, and the VL comprises the sequence as shown in any one of SEQ ID NOs: 16-18 and 60-63;
the VH comprises the sequence as shown in SEQ ID NO: 56, and the VL comprises the sequence as shown in any one of SEQ ID NOs: 16-18 and 60-63;
the VH comprises the sequence as shown in SEQ ID NO: 57, and the VL comprises the sequence as shown in any one of SEQ ID NOs: 16-18 and 60-63;
the VH comprises the sequence as shown in SEQ ID NO: 58, and the VL comprises the sequence as shown in any one of SEQ ID NOs: 16-18 and 60-63; or,
the VH comprises the sequence as shown in SEQ ID NO: 59, and the VL comprises the sequence as shown in any one of SEQ ID NOs: 16-18 and 60-63.

5. The antibody or antigen-binding fragment thereof according to any one of claims 1-4, wherein the VH and VL are selected from the group consisting of:
the VH and VL respectively comprise or are the sequences as shown in SEQ ID NOs: 1 and 3;
the VH and VL respectively comprise or are the sequences as shown in SEQ ID NOs: 2 and 4;
the VH and VL respectively comprise or are the sequences as shown in SEQ ID NOs: 1 and 5;
the VH and VL respectively comprise or are the sequences as shown in SEQ ID NOs: 6 and 9;
the VH and VL respectively comprise or are the sequences as shown in SEQ ID NOs: 7 and 10;
the VH and VL respectively comprise or are the sequences as shown in SEQ ID NOs: 7 and 11;
the VH and VL respectively comprise or are the sequences as shown in SEQ ID NOs: 8 and 11;
the VH and VL respectively comprise or are the sequences as shown in SEQ ID NOs: 8 and 51;
the VH and VL respectively comprise or are the sequences as shown in SEQ ID NOs: 8 and 52;
the VH and VL respectively comprise or are the sequences as shown in SEQ ID NOs: 8 and 53;
the VH and VL respectively comprise or are the sequences as shown in SEQ ID NOs: 48 and 54;
the VH and VL respectively comprise or are the sequences as shown in SEQ ID NOs: 49 and 52;
the VH and VL respectively comprise or are the sequences as shown in SEQ ID NOs: 50 and 51;
the VH and VL respectively comprise or are the sequences as shown in SEQ ID NOs: 50 and 55;
the VH and VL respectively comprise or are the sequences as shown in SEQ ID NOs: 12 and 16;
the VH and VL respectively comprise or are the sequences as shown in SEQ ID NOs: 13 and 17;
the VH and VL respectively comprise or are the sequences as shown in SEQ ID NOs: 14 and 18;
the VH and VL respectively comprise or are the sequences as shown in SEQ ID NOs: 15 and 18;
the VH and VL respectively comprise or are the sequences as shown in SEQ ID NOs: 56 and 60;
the VH and VL respectively comprise or are the sequences as shown in SEQ ID NOs: 57 and 61;
the VH and VL respectively comprise or are the sequences as shown in SEQ ID NOs: 15 and 62;
the VH and VL respectively comprise or are the sequences as shown in SEQ ID NOs: 58 and 63;
the VH and VL respectively comprise or are the sequences as shown in SEQ ID NOs: 15 and 63;
the VH and VL respectively comprise or are the sequences as shown in SEQ ID NOs: 59 and 63; and,
the VH and VL respectively comprise or are the sequences as shown in SEQ ID NOs: 57 and 63.

6. The antibody or antigen-binding fragment thereof according to any one of claims 1-5, wherein the anti-MASP2 antibody further comprises a heavy chain constant region and a light chain constant region;
preferably, the heavy chain constant region is selected from a human IgG1 constant region, a human IgG2 constant region, a human IgG3 constant region, a human IgG4 constant region or a variant thereof, and the light chain constant region is selected from a human κ chain constant region, a human λ chain constant region or a variant thereof;
more preferably, the heavy chain constant region is selected from an IgG4 constant region having any one or more mutations of S228P, M428L, N434S, M252Y, S254T and T256E, all mutations are numbered according to EU numbering;
most preferably, the heavy chain constant region comprises a sequence as shown in any one of SEQ ID NOs: 19-21 or an amino acid sequence having at least 85%, 90%, 91%, 92%, 93%, 94%, 95%, 96%, 97%, 98%, 99% or 100% identity thereto, and the light chain constant region comprises a sequence as shown in any one of SEQ ID NOs: 22-23 or an amino acid sequence having at least 85%, 90%, 91%, 92%, 93%, 94%, 95%, 96%, 97%, 98%, 99% or 100% identity thereto.

7. The antibody or antigen-binding fragment thereof according to any one of claims 1-6, wherein the anti-MASP2 antibody comprises a heavy chain and a light chain,
(i) the heavy chain has an amino acid sequence having at least 85%, 90%, 91%, 92%, 93%, 94%, 95%, 96%, 97%, 98%, 99% or 100% identity to a sequence as shown in any one of SEQ ID NOs: 37, 39, 42, and 43, and/or the light chain has an amino acid sequence having at least 85%, 90%, 91%, 92%, 93%, 94%, 95%, 96%, 97%, 98%, 99% or 100% identity to a sequence as shown in any one of SEQ ID NOs: 38, 40, and 41;
(ii) the heavy chain has an amino acid sequence having at least 85%, 90%, 91%, 92%, 93%, 94%, 95%, 96%, 97%, 98%, 99% or 100% identity to a sequence as shown in any one of SEQ ID NOs: 24, 28, 31, 44, 67, 69, 70, 80, and 81, and/or the light chain has an amino acid sequence having at least 85%, 90%, 91%, 92%, 93%, 94%, 95%, 96%, 97%, 98%, 99% or 100% identity to a sequence as shown in any one of SEQ ID NOs: 25, 29, 30, 64, 65, 66, 68, and 71; or
(iii) the heavy chain has an amino acid sequence having at least 85%, 90%, 91%, 92%, 93%, 94%, 95%, 96%, 97%, 98%, 99% or 100% identity to a sequence as shown in any one of SEQ ID NOs: 26, 32, 34, 36, 45, 72, 74, 77, and 79, and/or the light chain has an amino acid sequence having at least 85%, 90%, 91%, 92%, 93%, 94%, 95%, 96%, 97%, 98%, 99% or 100% identity to a sequence as shown in any one of SEQ ID NOs: 27, 33, 35, 73, 75, 76, and 78.

8. The antibody or antigen-binding fragment thereof according to any one of claims 1-7, wherein the anti-MASP2 antibody comprises a heavy chain and a light chain, wherein the heavy chain comprises the sequence as shown in SEQ ID NO: 37 and the light chain comprises the sequence as shown in any one of SEQ ID NOs: 38, 40, and 41;
the heavy chain comprises the sequence as shown in SEQ ID NO: 39 and the light chain comprises the sequence as shown in any one of SEQ ID NOs: 38, 40, and 41;
the heavy chain comprises the sequence as shown in SEQ ID NO: 42 and the light chain comprises the sequence as shown in any one of SEQ ID NOs: 38, 40, and 41;
the heavy chain comprises the sequence as shown in SEQ ID NO: 43 and the light chain comprises the sequence as shown in any one of SEQ ID NOs: 38, 40, and 41;
the heavy chain comprises the sequence as shown in SEQ ID NO: 24 and the light chain comprises the sequence as shown in any one of SEQ ID NOs: 25, 29, 30, 64, 65, 66, 68, and 71;
the heavy chain comprises the sequence as shown in SEQ ID NO: 28 and the light chain comprises the sequence as shown in any one of SEQ ID NOs: 25, 29, 30, 64, 65, 66, 68, and 71;
the heavy chain comprises the sequence as shown in SEQ ID NO: 31 and the light chain comprises the sequence as shown in any one of SEQ ID NOs: 25, 29, 30, 64, 65, 66, 68, and 71;
the heavy chain comprises the sequence as shown in SEQ ID NO: 44 and the light chain comprises the sequence as shown in any one of SEQ ID NOs: 25, 29, 30, 64, 65, 66, 68, and 71;
the heavy chain comprises the sequence as shown in SEQ ID NO: 67 and the light chain comprises the sequence as shown in any one of SEQ ID NOs: 25, 29, 30, 64, 65, 66, 68, and 71;
the heavy chain comprises the sequence as shown in SEQ ID NO: 69 and the light chain comprises the sequence as shown in any one of SEQ ID NOs: 25, 29, 30, 64, 65, 66, 68, and 71;
the heavy chain comprises the sequence as shown in SEQ ID NO: 70 and the light chain comprises the sequence as shown in any one of SEQ ID NOs: 25, 29, 30, 64, 65, 66, 68, and 71;
the heavy chain comprises the sequence as shown in SEQ ID NO: 80 and the light chain comprises the sequence as shown in any one of SEQ ID NOs: 25, 29, 30, 64, 65, 66, 68, and 71;
the heavy chain comprises the sequence as shown in SEQ ID NO: 81 and the light chain comprises the sequence as shown in any one of SEQ ID NOs: 25, 29, 30, 64, 65, 66, 68, and 71;
the heavy chain comprises the sequence as shown in SEQ ID NO: 26 and the light chain comprises the sequence as shown in any one of SEQ ID NOs: 27, 33, 35, 73, 75, 76, and 78;
the heavy chain comprises the sequence as shown in SEQ ID NO: 32 and the light chain comprises the sequence as shown in any one of SEQ ID NOs: 27, 33, 35, 73, 75, 76, and 78;
the heavy chain comprises the sequence as shown in SEQ ID NO: 34 and the light chain comprises the sequence as shown in any one of SEQ ID NOs: 27, 33, 35, 73, 75, 76, and 78;
the heavy chain comprises the sequence as shown in SEQ ID NO: 36 and the light chain comprises the sequence as shown in any one of SEQ ID NOs: 27, 33, 35, 73, 75, 76, and 78;
the heavy chain comprises the sequence as shown in SEQ ID NO: 45 and the light chain comprises the sequence as shown in any one of SEQ ID NOs: 27, 33, 35, 73, 75, 76, and 78;
the heavy chain comprises the sequence as shown in SEQ ID NO: 72 and the light chain comprises the sequence as shown in any one of SEQ ID NOs: 27, 33, 35, 73, 75, 76, and 78;
the heavy chain comprises the sequence as shown in SEQ ID NO: 74 and the light chain comprises the sequence as shown in any one of SEQ ID NOs: 27, 33, 35, 73, 75, 76, and 78;
the heavy chain comprises the sequence as shown in SEQ ID NO: 77 and the light chain comprises the sequence as shown in any one of SEQ ID NOs: 27, 33, 35, 73, 75, 76, and 78; or,
the heavy chain comprises the sequence as shown in SEQ ID NO: 79 and the light chain comprises the sequence as shown in any one of SEQ ID NOs: 27, 33, 35, 73, 75, 76, and 78.

9. The antibody or antigen-binding fragment thereof according to any one of claims 1-8, wherein the heavy chain and light chain respectively comprise sequences set forth in a pair selected from the group consisting of: sequences as shown in SEQ ID NOs: 24 and 25; sequences as shown in SEQ ID NOs: 26 and 27; sequences as shown in SEQ ID NOs: 28 and 29; sequences as shown in SEQ ID NOs: 28 and 30; sequences as shown in SEQ ID NOs: 31 and 30; sequences as shown in SEQ ID NOs: 31 and 64; sequences as shown in SEQ ID NOs: 31 and 65; sequences as shown in SEQ ID NOs: 31 and 66; sequences as shown in SEQ ID NOs: 67 and 68; sequences as shown in SEQ ID NOs: 69 and 65; sequences as shown in SEQ ID NOs: 70 and 64; sequences as shown in SEQ ID NOs: 70 and 71; sequences as shown in SEQ ID NOs: 32 and 33; sequences as shown in SEQ ID NOs: 34 and 35; sequences as shown in SEQ ID NOs: 36 and 35; sequences as shown in SEQ ID NOs: 37 and 38; sequences as shown in SEQ ID NOs: 39 and 40; sequences as shown in SEQ ID NOs: 37 and 41; sequences as shown in SEQ ID NOs: 72 and 73; sequences as shown in SEQ ID NOs: 74 and 75; sequences as shown in SEQ ID NOs: 36 and 76; sequences as shown in SEQ ID NOs: 77 and 78; sequences as shown in SEQ ID NOs: 36 and 78; sequences as shown in SEQ ID NOs: 79 and 78; sequences as shown in SEQ ID NOs: 74 and 78; sequences as shown in SEQ ID NOs: 42 and 38; sequences as shown in SEQ ID NOs: 43 and 38; sequences as shown in SEQ ID NOs: 44 and 30; sequences as shown in SEQ ID NOs: 44 and 64; sequences as shown in SEQ ID NOs: 44 and 65; sequences as shown in SEQ ID NOs: 44 and 66; sequences as shown in SEQ ID NOs: 80 and 68; sequences as shown in SEQ ID NOs: 81 and 65; and sequences as shown in SEQ ID NOs: 45 and 35.

10. An anti-MASP2 antibody or antigen-binding fragment thereof, which binds to or competes for binding to the same epitope as the anti-MASP2 antibody or antigen-binding fragment thereof according to any one of claims 1-9.

11. The anti-MASP2 antibody or antigen-binding fragment thereof according to any one of claims 1-10, which has at least one of the following characteristics:
(1) the antibody or antigen-binding fragment thereof binds to human MASP2 with a KD of 10 nM or lower;
(2) the antibody or antigen-binding fragment thereof inhibits complement lectin pathway MAC deposition in an *in vitro* assay with an IC₅₀ of less than 1, less than 0.9, less than 0.8, less than 0.7, less than 0.6, less than 0.5, less than 0.4, less than 0.3, less than 0.2, less than 0.1, less than 0.09, less than 0.08, less than 0.07, less than 0.06, less than 0.05, less than 0.04, less than 0.03, less than 0.02, less than 0.01 µg/mL or lower;
(3) the antibody or antigen-binding fragment thereof substantially does not inhibit the classical pathway and/or the alternative pathway;
(4) the antibody or antigen-binding fragment thereof substantially does not bind to complement components C1r, C1s, MASP1, and/or MASP3;
(5) when binding to human MASP2, the KD of the antibody or antigen-binding fragment thereof is substantially the same as or better than that of a reference MASP2 antibody;
(6) when binding to human MASP2, the antibody or antigen-binding fragment thereof specifically inhibits or blocks the complement lectin pathway, preferably, the inhibition or blocking effect of the antibody or antigen-binding fragment thereof is substantially the same as or better than that of a reference MASP2 antibody;
(7) when binding to human MASP2, the antibody or antigen-binding fragment thereof reduces or decreases C4b and/or MAC deposition, preferably, the antibody or antigen-binding fragment thereof reduces or decreases C4b and/or MAC deposition at a level substantially the same as or better than that of a reference MASP2 antibody.

12. The anti-MASP2 antibody or antigen-binding fragment thereof according to any one of claims 1-11, further comprising one or more effector molecules, preferably, the effector molecule is selected from an anti-tumor agent, a drug, a toxin, a bioactive protein, another antibody or antibody fragment, a synthetic or naturally occurring polymer, a nucleic acid and fragment thereof, a radionuclide, a radioisotope, a chelated metal, a nanoparticle and a reporter group, and a compound detectable by NMR or ESR spectroscopy.

13. A bispecific or multispecific antibody or antigen-binding fragment thereof, comprising the anti-MASP2 antibody or antigen-binding fragment thereof according to any one of claims 1-11.

14. A polynucleotide encoding the anti-MASP2 antibody or antigen-binding fragment thereof according to any one of claims 1-12, or encoding the bispecific or multispecific antibody or antigen-binding fragment thereof according to claim 13.

15. An expression vector comprising the polynucleotide according to claim 14.

16. A host cell comprising the polynucleotide according to claim 14 or the expression vector according to claim 15.

17. A pharmaceutical composition comprising the anti-MASP2 antibody or antigen-binding fragment thereof according to any one of claims 1-12, or the bispecific or multispecific antibody or antigen-binding fragment thereof according to claim 13, or the polynucleotide according to claim 14, or the expression vector according to claim 15, or the host cell according to claim 16, and one or more pharmaceutically acceptable carriers.

18. A kit comprising the anti-MASP2 antibody or antigen-binding fragment thereof according to any one of claims 1-12.

19. A method for preventing and/or treating a disease or disorder associated with MASP2 in a subject, comprising administering a therapeutically or prophylactically effective amount of the anti-MASP2 antibody or antigen-binding fragment thereof according to any one of claims 1-12, the bispecific or multispecific antibody or antigen-binding fragment thereof according to claim 13, the polynucleotide according to claim 14, the expression vector according to claim 15, the host cell according to claim 16, the pharmaceutical composition according to claim 17, or the kit according to claim 18 to a subject in need thereof; preferably, the disease or disorder associated with MASP2 is IgA nephropathy.
